# EUROPEAN PATENT SPECIFICATION

(11) **EP 2 854 528 B1**
(45) Date of publication and mention of the grant of the patent: **18.09.2019**
(21) Application number: 13797642.9
(22) Date of filing: 31.05.2013
(51) Int. Cl.: A61K 31/122, A61K 9/19, A61K 9/10, A61P 35/00, A61K 45/06, A61K 47/22, A61K 47/24, A61K 47/44, A61K 31/7068

(54) **TREATMENT OF SOLID TUMORS USING COENZYME Q10**
BEHANDLUNG VON FESTEN TUMOREN MITTELS DES COENZYMS Q10
TRAITEMENT DE TUMEURS SOLIDES AU MOYEN DE LA COENZYME Q10

(30) Priority: 01.06.2012 US 201261654245 P
(43) Date of publication of application: 08.04.2015
(73) Proprietor: Berg LLC, Nashville, TN 37210 (US)
(72) Inventor: NARAIN, Niven, Rajin, Cambridge, MA 02138 (US); MCCOOK, John, Patrick, Frisco, TX 75034 (US); SONG, Paul, Y., Santa Monica, CA 90402 (US)
(74) Representative: Hoffmann Eitle
(86) International application number: PCT/US2013/043785
(87) International publication number: WO 2013/181639

(56) References cited:
- EP-A1- 2 371 363
- WO-A2-2011/112900
- US-A1- 2010 003 192
- US-A1- 2011 027 247
- US-B2- 8 147 825
- LOCKWOOD K ET AL: "Progress on therapy of breast cancer with vitamin Q10 and the regression of metastases", BIOCHEMICAL AND BIOPHYSICAL RESEARCH COMMUNICATIONS, ACADEMIC PRESS INC. ORLANDO, FL, US, vol. 212, no. 1, 1 January 1995 (1995-01-01), pages 172-177, XP002286385, ISSN: 0006-291X, DOI: 10.1006/BBRC.1995.1952
- FOLKERS K ET AL: "Survival of Cancer Patients on Therapy with Coenzyme Q10", BIOCHEMICAL AND BIOPHYSICAL RESEARCH COMMUNICATIONS, ACADEMIC PRESS INC. ORLANDO, FL, US, vol. 192, no. 1, 15 April 1993 (1993-04-15), pages 241-245, XP024768101, ISSN: 0006-291X, DOI: 10.1006/BBRC.1993.1405 [retrieved on 1993-04-15]
- LOCKWOOD K ET AL: "Partial and Complete Regression of Breast Cancer in Patients in Relation to Dosage of Coenzyme Q10", BIOCHEMICAL AND BIOPHYSICAL RESEARCH COMMUNICATIONS, ACADEMIC PRESS INC. ORLANDO, FL, US, vol. 199, no. 3, 31 March 1994 (1994-03-31), pages 1504-1508, XP024764919, ISSN: 0006-291X, DOI: 10.1006/BBRC.1994.1401 [retrieved on 1994-03-31]
- GALILI ET AL: "Clinical response of myelodysplastic syndromes patients to treatment with coenzyme Q10", LEUKEMIA RESEARCH, NEW YORK,NY, US, vol. 31, no. 1, 11 November 2006 (2006-11-11), pages 19-26, XP005762136, ISSN: 0145-2126, DOI: 10.1016/J.LEUKRES.2006.04.009

## Description

### RELATED APPLICATIONS

This application claims priority to U.S. Provisional Patent Application No. 61/654,245 filed on June 1, 2012.

### BACKGROUND

Cancer is presently one of the leading causes of death in developed nations. A diagnosis of cancer traditionally involves serious health complications. Cancer can cause disfigurement, chronic or acute pain, lesions, organ failure, or even death. Commonly diagnosed cancers include pancreatic cancer, breast cancer, lung cancer, melanoma, lymphoma, carcinoma, sarcoma non-Hodgkin's lymphoma, leukemia, endometrial cancer, colon and rectal cancer, prostate cancer, and bladder cancer. Traditionally, many cancers (e.g., breast cancer, leukemia, lung cancer, or the like) are treated with surgery, chemotherapy, radiation, or combinations thereof. Chemotherapeutic agents used in the treatment of cancer are known to produce several serious and unpleasant side effects in patients. For example, some chemotherapeutic agents cause neuropathy, nephrotoxicity (e.g., hyperlipidemia, proteinuria, hypoproteinemia, combinations thereof, or the like), stomatitis, mucositisemesis, alopecia, anorexia, esophagitis amenorrhoea, decreased immunity, anaemia, high tone hearing loss, cardiotoxicity, fatigue, neuropathy, or combinations thereof. Oftentimes, chemotherapy is not effective, or loses effectiveness after a period of efficacy, either during treatment, or shortly after the treatment regimen concludes (i.e., the treatment regimen does not result in a cure). Improved methods for the treatment of oncological diseases, including cancer, and composition capable of delivering bioactive agents to aid in the treatment of diseases and other conditions remain desirable.

Methods for the treatment of oncological diseases involving the use of Coenzyme Q10 are described in the following literature: LOCKWOOD KET AL, "Progress on therapy of breast cancer with vitamin Q10 and the regression of metastases", BIOCHEMICAL AND BIOPHYSICAL RESEARCH COMMUNICATIONS, ACADEMIC PRESS INC. ORLANDO, FL, US, (19950101), vol. 212, no. 1, doi:10.1006/BBRC.1995.1952, ISSN 0006-291X, pages 172 - 177; FOLKERS K ET AL, "Survival of Cancer Patients on Therapy with Coenzyme Q10", BIOCHEMICAL AND BIOPHYSICAL RESEARCH COMMUNICATIONS, ACADEMIC PRESS INC. ORLANDO, FL, US, vol. 192, no. 1, doi:10.1006/BBRC.1993.1405, ISSN 0006-291X, (19930415), pages 241 - 245, (19930415); EP 2 371 363 A; WO 20111/112900 A; and LOCKWOOD K ET AL, "Partial and Complete Regression of Breast Cancer in Patients in Relation to Dosage of Coenzyme Q10", BIOCHEMICAL AND BIOPHYSICAL RESEARCH COMMUNICATIONS, ACADEMIC PRESS INC. ORLANDO, FL, US, vol. 199, no. 3, doi:10.1006/BBRC.1994.1401, ISSN 0006-291X, (19940331), pages 1504 - 1508.

### SUMMARY OF THE INVENTION

The invention provides compositions for treatment of a cancer in a subject by administering coenzyme Q10 (CoQ10) wherein the subject has failed at least one (e.g., 1, 2, 3, 4, 5, 6, 7, 8, 9, 10) prior chemotherapeutic regimen for that cancer as specified in appended claim 1.

In certain embodiments of the invention, as specified in appended claim 1, the subject has failed treatment for the cancer with at least two previous chemotherapeutic regimens. That is, in certain embodiments, the subject has failed 2, 3, 4, 5, 6, 7, 8, 9 10 or more treatment regimens. In certain embodiments, the subject has failed treatment for the cancer with at least three previous chemotherapeutic regimens. In certain embodiments, the subject has failed treatment for the cancer with at least four previous chemotherapeutic regimens. In certain embodiments, the subject has failed treatment for the cancer with at least five previous chemotherapeutic regimens.

In certain embodiments of the invention, as specified in appended claim 1, the cancer is a refractory cancer.

In certain embodiments of the invention, as specified in appended claim 1, failure with at least one chemotherapeutic regimen comprises tumor growth during or after treatment with the chemotherapeutic regimen. In certain embodiments of the invention, failure with at least one chemotherapeutic regimen comprises a dose limiting toxicity due to the chemotherapeutic regimen. In certain embodiments of the invention, failure with at least one chemotherapeutic regimen comprises a grade IV toxicity due to the chemotherapeutic regimen.

In certain embodiments of the invention, as specified in appended claim 1, the subject demonstrates a clinical benefit as a result of administration of the CoQ10 compound. For example, in certain embodiments, the clinical benefit is one or more clinical benefits selected from the group consisting of stable disease per RECIST 1.1 criteria, partial response per RECIST 1.1 criteria, and complete response per RECIST 1.1 criteria.

In certain embodiments of the invention, as specified in appended claim 1, the subject does not exhibit a dose limiting toxicity in response as a result of administration of the CoQ10 compound. For example, the subject does not exhibit a grade III toxicity as a result of administration of the CoQ10 compound. In certain embodiments, the subject does not exhibit a grade IV toxicity as a result of administration of the CoQ10 compound.

In certain embodiments of the invention, as specified in appended claim 1, the cancer comprises a Stage III tumor. In certain embodiments, the cancer comprises a Stage IV tumor. In certain embodiments, the cancer is metastatic. In certain embodiments, the cancer comprises a solid tumor. In certain embodiments, the cancer is selected from the group consisting of a sarcoma, a carcinoma and a melanoma.

In the methods of the invention as specified in appended claim 1, the cancer is a cancer selected from the group consisting of colon cancer, rectal cancer, pancreatic cancer, liver cancer, myxoid liposarcoma, oral cancer and uterine cancer.

In certain embodiments of the invention, as specified in appended claim 1, the subject has failed treatment with one or more of adriamycin, ifosfamide, etoposide, vincristine, gemzar, taxotere, and Th-302. In certain embodiments of the invention, the subject has failed treatment with one or more of a topoisomerase I inhibitor, a topoisomerase II inhibitor, a mitotic inhibitor, an alkylating agent, a platinum compound, and an antimetabolite.

In certain embodiments of the invention, as specified in appended claim 1, the CoQ10 compound is administered at least one time per week. In certain embodiments, the CoQ10 compound is administered at least two times per week. In certain embodiments, the CoQ10 compound is administered at least three times per week. In certain embodiments, the CoQ10 compound is administered one time per week. In certain embodiments, the CoQ10 compound is administered two times per week. In certain embodiments, the CoQ10 compound is administered three times per week.

In certain embodiments of the invention, as specified in appended claim 1, the CoQ10 compound is administered at a dose selected from the group consisting of at least 5.6 mg/kg/dose, at least 11.2 mg/kg/dose, at least 22.5 mg/kg/dose, at least 33 mg/kg/dose, at least 44 mg/kg/dose, at least 58.7 mg/kg/dose, at least 78.2 mg/kg/dose, at least 104.3 mg/kg/dose, and at least 139 mg/kg/dose. In certain embodiments, the CoQ10 compound is administered at a dose of at least 50 mg/kg/dose, at least 75 mg/kg/dose, at least 100 mg/kg/dose, at least 125 mg/kg/dose, at least 150 mg/kg/dose, at least 200 mg/kg/dose.

In certain embodiments of the invention, as specified in appended claim 1, the CoQ10 compound is administered at a dose of no more than 500 mg/kg/dose, no more than 400 mg/kg/dose, no more than 300 mg/kg/dose.

In certain embodiments of the invention, as specified in appended claim 1, the CoQ10 compound is administered at a dose that does not result in a Grade III toxicity in the subject. In certain embodiments, the CoQ10 compound is administered at a dose that does not result in a Grade IV toxicity to the subject.

In certain embodiments of the invention, as specified in appended claim 1, at least 12 doses of the CoQ10 compound are administered to the subject. That is, in certain embodiments, at least 8, 9, 10, 11, 12, 13, 14, 15, 16, 17, 18, 19, 20, 21, 22, 23, 24, 25, 62, 27, 28, 29, 30, 31, 32, 33, 34, 35, 36, 37, 38, 39, 40, 45, 50, 55, 60, 65, 70, 75, 80, 85, 90, 95, 100, or more doses are administered to the subject.

In certain embodiments of the invention, as specified in appended claim 1, the subject is treated with CoQ10 for at least 4 weeks. In certain embodiments, the subject is treated with CoQ10 for at least 8 weeks. That is, in certain embodiments, the subject is treated with CoQ10 for at least 4, 5, 6, 7, 8, 9, 10, 11, 12, 13, 14, 15, 16, 17, 18, 19, 20, 21, 22, 23, 24, 25, 62, 27, 28, 29, 30, 31, 32, 33, 34, 35, 36, 37, 38, 39, 40, 45, 50, 55, 60, 65, 70, 75, 80, 85, 90, 95, 100, or more weeks.

In certain embodiments of the invention, as specified in appended claim 1, the subject has been treated with fewer than 8 prior chemotherapeutic regimens. That is, in certain embodiments, the subject has been treated with 8, 7, 6, 5, 4, 3, 2, or 1 prior chemotherapeutic regimens.

In certain embodiments of the invention, as specified in appended claim 1, the CoQ10 compound is administered by injection or infusion. In certain embodiments of the invention, the CoQ10 compound is administered intravenously. In certain embodiments of the invention, the CoQ10 compound is administered topically. In certain embodiments of the invention, the CoQ10 compound is administered by inhalation.

In certain embodiments of the invention, as specified in appended claim 1, the subject is human.

In certain embodiments of the invention, as specified in appended claim 1, the CoQ10 compound is formulated as a nanodispersion.

In certain embodiments of the invention, as specified in appended claim 1, the CoQ10 compound is provided for intravenous administration in a CoQ10 formulation comprising:
an aqueous solution;
a CoQ10 dispersed into a nano-dispersion of particles; and
at least one of a dispersion stabilizing agent and an opsonization reducer;
wherein the nano-dispersion of the CoQ10 is dispersed into nano-particles having a mean particle size of less than 200-nm. In certain embodiments, the dispersion stabilizing agent is selected from the group consisting of pegylated castor oil, Cremophor® EL, Cremophor® RH 40, Pegylated vitamin E, Vitamin E TPGS, and Dimyristoylphosphatidyl choline (DMPC). In certain embodiments, the dispersion stabilizing agent is DMPC. In certain embodiments, the opsonization reducer is selected from the group consisting of poloxamers and poloxamines. In certain embodiments, the opsonization reducer is poloxamer 188. In certain embodiments, the opsonization reducer is poloxamer 188 and the dispersion stabilizing agent is DMPC.

In certain embodiments of the invention, as specified in appended claim 1, the CoQ10 formulation has a weight-per-volume of the CoQ10, DMPC and poloxamer 188 of 4%, 3% and 1.5%, respectively.

In certain embodiments of the invention, as specified in appended claim 1, the CoQ10 compound is administered to the subject with an additional agent. In certain embodiments, additional agent is a chemotherapeutic agent.

Other embodiments are provided *infra.*

### BRIEF DESCRIPTION OF THE DRAWINGS

Various embodiments of the present disclosure will be described herein below with reference to the figures wherein:
Figure 1 shows a progression free survival curve from the Phase 1 trial described herein.
Figures 2A and B show CT images of a 62-year-old woman with myxoid liposarcoma with metastatic disease to the mediastinum, heart, and lungs (A) before and (B) after treatment with 4 cycles of coenzyme Q10 at a dose of 56.8 mg/kg/dose. The tumor measurements are indicated in Figure 2A.
Figure 3A and B show CT images of a 62-year-old woman with pleomorphic fibrosarcoma of the left ilium with diffuse bone metastasis (A) before and (B) after treatment with 6 cycles of coenzyme Q10. The tumor measurements are indicated in Figures 3A and B.
Figure 4 illustrates the half-life and the Cₘₐₓ and Tₘₐₓ of CoQ10 in the C31510 formulation associated with the end of the infusion.
Figure 5 shows the dose-proportionality of coenzyme Q10 in the C31510 formulation. For AUC, the dose-proportionality is not strictly proportional to dose, as seen by the intercept not going close to the origin.
Figure 6 shows that the Cₘₐₓ for CoQ10 administered in the C31510 formulation and the dosage provided is only related to maximum exposure, not overall exposure.

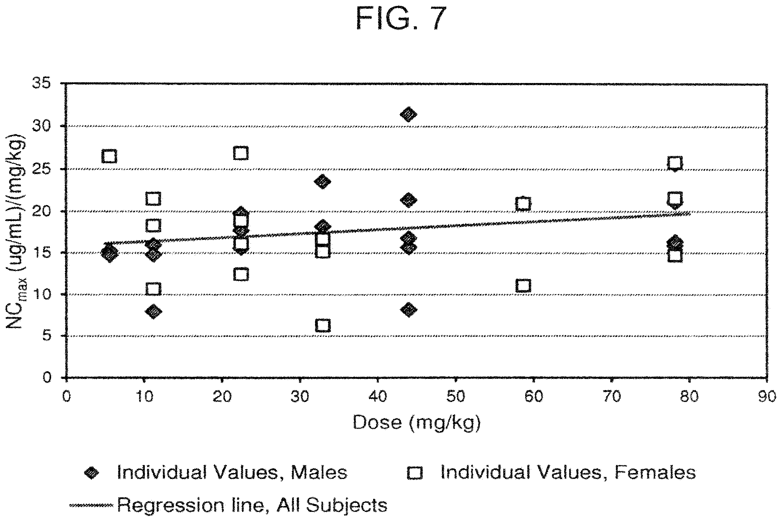

Figure 7 shows the lack of difference in pharmacokinetics between males and females.

### DETAILED DESCRIPTION AND PREFERRED EMBODIMENTS

### I. Definitions

The terms "cancer" or "tumor" are well known in the art and refer to the presence, *e.g.,* in a subject, of cells possessing characteristics typical of cancer-causing cells, such as uncontrolled proliferation, immortality, metastatic potential, rapid growth and proliferation rate, decreased cell death/apoptosis, and certain characteristic morphological features.

As used herein, "cancer" refers to all types of cancer or neoplasm or malignant tumors found in humans, including, but not limited to: leukemias, lymphomas, melanomas, carcinomas and sarcomas. As used herein, the terms or language "cancer," "neoplasm," and "tumor," are used interchangeably and in either the singular or plural form, refer to cells that have undergone a malignant transformation that makes them pathological to the host organism. Primary cancer cells (that is, cells obtained from near the site of malignant transformation) can be readily distinguished from non-cancerous cells by well-established techniques, particularly histological examination. The definition of a cancer cell, as used herein, includes not only a primary cancer cell, but also cancer stem cells, as well as cancer progenitor cells or any cell derived from a cancer cell ancestor. This includes metastasized cancer cells, and in vitro cultures and cell lines derived from cancer cells. In certain embodiments, the cancer is not a central nervous system (CNS) cancer, i.e., not a cancer of a tumor present in at least one of the spinal cord, the brain, and the eye. In certain embodiments, the primary cancer is not a CNS cancer.

A "solid tumor" is a tumor that is detectable on the basis of tumor mass; e.g., by procedures such as CAT scan, MR imaging, X-ray, ultrasound or palpation, and/or which is detectable because of the expression of one or more cancer-specific antigens in a sample obtainable from a patient. The tumor does not need to have measurable dimensions.

Specific criteria for the staging of cancer are dependent on the specific cancer type based on tumor size, histological characteristics, tumor markers, and other criteria known by those of skill in the art. Generally, cancer stages can be described as follows:
Stage 0 Carcinoma in situ
Stage I, Stage II, and Stage III Higher numbers indicate more extensive disease: Larger tumor size and/or spread of the cancer beyond the organ in which it first developed to nearby lymph nodes and/or tissues or organs adjacent to the location of the primary tumor
Stage IV The cancer has spread to distant tissues or organs

As used herein, the terms "treat," "treating" or "treatment" refer, preferably, to an action to obtain a beneficial or desired clinical result including, but not limited to, alleviation or amelioration of one or more signs or symptoms of a disease or condition (e.g., regression, partial or complete), diminishing the extent of disease, stability (*i.e.,* not worsening, achieving stable disease) state of disease, amelioration or palliation of the disease state, diminishing rate of or time to progression, and remission (whether partial or total). "Treatment" of a cancer can also mean prolonging survival as compared to expected survival in the absence of treatment. Treatment need not be curative. In certain embodiments, treatment includes one or more of a decrease in pain or an increase in the quality of life (QOL) as judged by a qualified individual, e.g., a treating physician, e.g., using accepted assessment tools of pain and QOL. In certain embodiments, treatment does not include one or more of a decrease in pain or an increase in the quality of life (QOL) as judged by a qualified individual, e.g., a treating physician, e.g., using accepted assessment tools of pain and QOL.

RECIST criteria are clinically accepted assessment criteria used to provide a standard approach to solid tumor measurement and provide definitions for objective assessment of change in tumor size for use in clinical trials. Such criteria can also be used to monitor response of an individual undergoing treatement for a solid tumor. The RECIST 1.1 criteria are discussed in detail in Eisenhauer et al., New response evaluation criteria in solid tumors: Revised RECIST guideline (version 1.1). Eur. J. Cancer. 45:228-247, 2009. Response criteria for target lesions include:
Complete Response (CR): Disappearance of all target lesions. Any pathological lymph nodes (whether target or non-target) must have a reduction in short axis to <10 mm.
Partial Response (PR): At least a 30% decrease in the sum of diameters of target lesion, taking as a reference the baseline sum diameters.
Progressive Diseases (PD): At least a 20% increase in the sum of diameters of target lesions, taking as a reference the smallest sum on the study (this includes the baseline sum if that is the smallest on the study). In addition to the relative increase of 20%, the sum must also demonstrate an absolute increase of at least 5 mm. (Note: the appearance of one or more new lesions is also considered progression.)
Stable Disease (SD): Neither sufficient shrinkage to qualify for PR nor sufficient increase to qualify for PD, taking as a reference the smallest sum diameters while on study.

RECIST 1.1 criteria also consider non-target lesions which are defined as lesions that may be measureable, but need not be measured, and should only be assessed qualitatively at the desired time points. Response criteria for non-target lesions include:
Complete Response (CR): Disappearance of all non-target lesions and normalization of tumor marker levels. All lymph nodes must be non-pathological in size (< 10 mm short axis).
Non-CR/ Non-PD: Persistence of one or more non-target lesion(s) and/ or maintenance of tumor marker level above the normal limits.
Progressive Disease (PD): *Unequivocal progression* (emphasis in original) of existing non-target lesions. The appearance of one or more new lesions is also considered progression. To achieve "unequivocal progression" on the basis of non-target disease, there must be an overall level of substantial worsening of non-target disease such that, even in the presence of SD or PR in target disease, the overall tumor burden has increased sufficiently to merit discontinuation of therapy. A modest "increase" in the size of one or more non-target lesions is usually not sufficient to qualify for unequivocal progression status. The designation of overall progression solely on the basis of change in non-target disease in the face of SD or PR in target disease will therefore be extremely rare.

"Chemotherapeutic agent" is understood as a drug used for the treatment of cancer. Chemotherapeutic agents include, but are not limited to, small molecules, hormones and hormone analogs, and biologics (e.g., antibodies, peptide drugs, nucleic acid drugs).

A "chemotherapeutic regimen" is a clinically accepted dosing protocol for the treatment of cancer that includes administration of one or more chemotherapeutic agents to a subject in specific amounts on a specific schedule.

A "subject who has failed a chemotherapeutic regimen" is a subject with cancer that does not respond, or ceases to respond to treatment with a chemotherapeutic regimen per RECIST 1.1 criteria (see, Eisenhauer et al., 2009 and as discussed above), i.e., does not achieve a complete response, partial response, or stable disease in the target lesion; or does not achieve complete response or non-CR/non-PD of non-target lesions, either during or after completion of the chemotherapeutic regimen, either alone or in conjunction with surgery and/or radiation therapy which, when possible, are often clinically indicated in conjunction with chemotherapy. A failed chemotherapeutic regime results in, e.g., tumor growth, increased tumor burden, and/ or tumor metastasis. A failed chemotherapeutic regimen as used herein includes a treatment regimen that was terminated due to a dose limiting toxicity, e.g., a grade III or a grade IV toxicity that cannot be resolved to allow continuation or resumption of treatment with the chemotherapeutic agent or regimen that caused the toxicity. A failed chemotherapeutic regimen includes a treatment regimen that does not result in at least stable disease for all target and non-target lesions for an extended period, e.g., at least 1 month, at least 2 months, at least 3 months, at least 4 months, at least 5 months, at least 6 months, at least 12 months, at least 18 months, or any time period less than a clinically defined cure. A failed chemotherapeutic regimen includes a treatment regimen that results in progressive disease of at least one target lesion during treatment with the chemotherapeutic agent, or results in progressive disease less than 2 weeks, less than 1 month, less than two months, less than 3 months, less than 4 months, less than 5 months, less than 6 months, less than 12 months, or less than 18 months after the conclusion of the treatment regimen, or less than any time period less than a clinically defined cure.

A failed chemotherapeutic regimen does not include a treatment regimen wherein the subject treated for a cancer achieves a clinically defined cure, e.g., 5 years of complete response after the end of the treatment regimen, and wherein the subject is subsequently diagnosed with a distinct cancer, e.g., more than 5 years, more than 6 years, more than 7 years, more than 8 years, more than 9 years, more than 10 years, more than 11 years, more than 12 years, more than 13 years, more than 14 years, or more than 15 years after the end of the treatment regimen. For example, a subject who suffered from a pediatric cancer may develop cancer later in life after being cured of the pediatric cancer. In such a subject, the chemotherapeutic regimen to treat the pediatric cancer is considered to have been successful.

A "refractory cancer" is a malignancy for which surgery is ineffective, which is either initially unresponsive to chemo- or radiation therapy, or which becomes unresponsive to chemo- or radiation therapy over time.

A "therapeutically effective amount" is that amount sufficient to treat a disease in a subject. A therapeutically effective amount can be administered in one or more administrations.

The terms "administer", "administering" or "administration" include any method of delivery of a pharmaceutical composition or agent into a subject's system or to a particular region in or on a subject. In certain embodiments, the agent is delivered orally. In certain embodiments, the agent is administered parenterally. In certain embodiments, the agent is delivered by injection or infusion. In certain embodiments, the agent is delivered topically including transmucosally. In certain embodiments, the agent is delivered by inhalation. In certain embodiments of the invention, an agent is administered by parenteral delivery, including, intravenous, intramuscular, subcutaneous, intramedullary injections, as well as intrathecal, direct intraventricular, intraperitoneal, intranasal, or intraocular injections. In one embodiment, the compositions provided herein may be administered by injecting directly to a tumor. In some embodiments, the formulations of the invention may be administered by intravenous injection or intravenous infusion. In certain embodiments, the formulation of the invention can be administered by continuous infusion. In certain embodiments, administration is not oral. In certain embodiments, administration is systemic. In certain embodiments, administration is local. In some embodiments, one or more routes of administration may be combined, such as, for example, intravenous and intratumoral, or intravenous and peroral, or intravenous and oral, intravenous and topical, or intravenous and transdermal or transmucosal. Administering an agent can be performed by a number of people working in concert. Administering an agent includes, for example, prescribing an agent to be administered to a subject and/or providing instructions, directly or through another, to take a specific agent, either by self-delivery, e.g., as by oral delivery, subcutaneous delivery, intravenous delivery through a central line, etc.; or for delivery by a trained professional, e.g., intravenous delivery, intramuscular delivery, intratumoral delivery, etc.

As used herein, "continuous infusion" is understood as administration of a dose of the formulation continuously for at least 24 hours. Continuous administration is typically facilitated by use of a pump, either an implantable or external pump. A formulation can be administered by continuous infusion in multiple, separated doses, with a break of one or more days between continuous infusion doses.

As used herein, a "pharmaceutically acceptable" component is one that is suitable for use with humans and/or animals without undue adverse side effects (such as toxicity, irritation, and allergic response) commensurate with a reasonable benefit/risk ratio.

As used herein, a "formulation" is understood as an active ingredient, e.g., CoQ10, a metabolite of CoQ10, a biosynthetic precursor of CoQ10, or a CoQ10 related compound, in combination with any pharmaceutically acceptable carrier. Formulations can include, but are not limited to, aqueous formulations, liposomal formulations, suspensions, emulsions, microemulsions, nanoemulsions, nanosuspensions, formulations for specific routes of administration, such as cream, lotion, and ointment formulations for topical administration, solid formulations for oral administration, and liquid formulations for injection or inhalation.

As used herein, the term "safe and therapeutic effective amount" refers to the quantity of a component which is sufficient to yield a desired therapeutic response without undue adverse side effects (such as toxicity, irritation, or allergic response) commensurate with a reasonable benefit/risk ratio when used in the manner of this disclosure. By "therapeutically effective amount" is meant an amount of a compound of the present disclosure effective to yield the desired therapeutic response. The specific safe and effective amount or therapeutically effective amount will vary with such factors as the particular condition being treated, the physical condition of the patient, the type of mammal or animal being treated, the duration of the treatment, the nature of concurrent therapy (if any), and the specific formulations employed and the structure of the compounds or its derivatives. The "therapeutically effective amount" will vary depending on the compound, the disease and its severity and the age, weight, etc., of the patient to be treated.

The term "therapeutic effect" refers to a local or systemic effect in animals, particularly mammals, and more particularly humans caused by a pharmacologically active substance. The term thus means any substance intended for use in the diagnosis, cure, mitigation, treatment or prevention of disease or in the enhancement of desirable physical or mental development and conditions in an animal or human. The phrase "therapeutically-effective amount" means that amount of such a substance that produces some desired local or systemic effect at a reasonable benefit/risk ratio applicable to any treatment. In certain embodiments, a therapeutically-effective amount of a compound will depend on its therapeutic index, solubility, and the like.

"Adverse events" or "AEs" are characterized by grade depending on the severity. Some AE (e.g., nausea, low blood counts, pain, reduced blood clotting) can be treated so that the specific chemotherapeutic regimen can be continued or resumed. Some adverse events (e.g., loss of cardiac, liver, or kidney function; nausea) may not be treatable, requiring termination of treatment with the drug. Determination of AE grade and appropriate interventions can be determined by those of skill in the art. Common Terminology Criteria for Adverse Events v4.0 (CTCAE) (Publish Date: May 28, 2009) provide a grading scale for adverse events as follows:
Grade 1 Mild; asymptomatic or mild symptoms; clinical or diagnostic observations only; intervention not indicated.
Grade 2 Moderate; minimal, local or noninvasive intervention indicated; limiting age-appropriate instrumental activities of daily life (ADL).
Grade 3 Severe or medically significant but not immediately life-threatening; hospitalization or prolongation of hospitalization indicated; disabling, limiting self care ADL.
Grade 4 Life-threatening consequences; urgent intervention indicated.
Grade 5 Death related to adverse event.

As used herein, "co-administration" or "combination therapy" is understood as administration of two or more active agents using separate formulations or a single pharmaceutical formulation, or consecutive administration in any order such that, there is a time period while both (or all) active agents simultaneously exert their biological activities. Co-administration does not require that the agents are administered at the same time, at the same frequency, or by the same route of administration. As used herein, "co-administration" or "combination therapy" includes administration of a CoQ10 compound with one or more additional anti-cancer agents, e.g., chemotherapeutic agents, or administration of two or more CoQ10 compounds. Examples of anticancer agents, including chemotherapeutic agents, are provided herein.

As used herein, the term "survival" refers to the continuation of life of a subject which has been treated for a disease or condition, e.g., cancer. The time of survival can be defined from an arbitrary point such as time of entry into a clinical trial, time from completion or failure or an earlier treatment regimen, time from diagnosis, etc.

As used herein, the term "subject" refers to human and non-human animals, including veterinary subjects. The term "non-human animal" includes all vertebrates, *e.g*., mammals and non-mammals, such as non-human primates, mice, rabbits, sheep, dog, cat, horse, cow, chickens, amphibians, and reptiles. In a preferred embodiment, the subject is a human and may be referred to as a patient.

The articles "a", "an" and "the" are used herein to refer to one or to more than one (*i.e.* to at least one) of the grammatical object of the article unless otherwise clearly indicated by contrast. By way of example, "an element" means one element or more than one element.

The term "including" is used herein to mean, and is used interchangeably with, the phrase "including but not limited to".

The term "or" is used herein to mean, and is used interchangeably with, the term "and/or," unless context clearly indicates otherwise.

The term "such as" is used herein to mean, and is used interchangeably, with the phrase "such as but not limited to".

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1 %, 0.05%, or 0.01% of the stated value. Unless otherwise clear from context, all numerical values provided herein can be modified by the term about.

Ranges provided herein are understood to be shorthand for all of the values within the range. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

The recitation of a listing of chemical group(s) in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

### II. Coenzyme Q10 CoQ10 has the following structure:

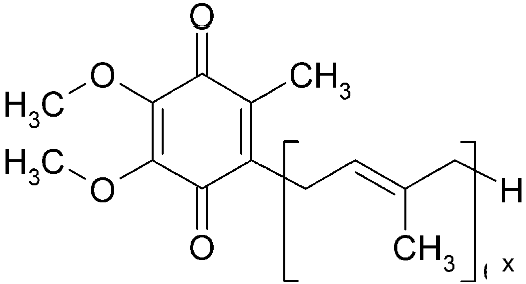

wherein x is 10. CoQ10 includes the fully oxidized version, also known as ubiquinone, the partially oxidized version, also known as semiquinone or ubisemiquinone, or the fully reduced version, also known as ubiquinol; or any mixtures or combinations thereof. In certain embodiments, the CoQ10 for treatment of cancer is ubiquinone. In certain embodiments, the CoQ10 for treatment of cancer is ubiquinol.

In the present invention, the therapeutic agent is Coenzyme Q10 (CoQ10). Coenzyme Q10, also referred to herein as CoQ10, is also known as ubiquinone, or ubidecarenone. CoQ10 is art-recognized and further described in International Publication No. WO 2005/069916 (Appln. No. PCT/US2005/001581), WO 2008/116135 (Appln. No. PCT/US08/57786), WO2010/132507 (Appln. No. PCT/US2010/034453), WO 2011/112900 (Appln. No. PCT/US2011/028042), and WO2012/174559 (Appln. No. PCT/US2012/043001). CoQ10 is one of a series of polyprenyl 2,3-dimethoxy-5-methylbenzoquinone (ubiquinone) present in the mitochondrial electron transport systems of eukaryotic cells. Human cells produce CoQ10 exclusively and it is found in cell and mitochondrial membranes of all human cells, with the highest levels in organs with high energy requirements, such as the liver and the heart. The body pool of CoQ10 has been estimated to be about 2 grams, of which more than 50% is endogenous. Approximately 0.5 grams of CoQ10 is required from the diet or biosynthesis each day. CoQ10 is produced in ton quantities from the worldwide supplement market and can be obtained from Kaneka, with plants in Pasadena, Texas and Takasagoshi, Japan.

Coenzyme Q10 related compounds include, but are not limited to, benzoquinones, isoprenoids, farnesols, farnesyl acetate, farnesyl pyrophosphate, 1-phenylalanine, d-phenylalanine, dl-phenylalanine, 1-tyrosine, d- tyrosine, dl-tyrosine, 4-hydroxy-phenylpyruvate, 4-hydroxy-phenyllactate, 4-hydroxy- cinnamate, dipeptides and tripeptides of tyrosine or phenylalanine, 3,4-dihydroxymandelate, 3- methoxy-4-hydroxyphenylglycol, 3-methoxy-4-hydroxymandelate, vanillic acid, phenylacetate, pyridoxine, S-adenosyl methionine, panthenol, mevalonic acid, isopentyl pyrophosphate, phenylbutyrate, 4-hydroxy-benzoate,decaprenyl pyrophosphate, beta-hydroxybutyrate, 3- hydroxy-3-methyl-glutarate, acetylcarnitine, acetoacetylcarnitine, acetylglycine, acetoacetylglycine, carnitine, acetic acid, pyruvic acid, 3-hydroxy-3-methylglutarylcarnitine, all isomeric forms of serine, alanine, cysteine, glycine, threonine, hydroxyproline, lysine, isoleucine, and leucine, even carbon number C4 to C8 fatty acids (butyric, caproic, caprylic, capric, lauric, myristic, palmitic, and stearic acids) salts of carnitine and glycine, e.g., palmitoylcarnitine and palmitoylglycine, and 4-hydroxy-benzoate polyprenyltransferase, any salts of these compounds, as well as any combinations thereof, and the like.

Metabolites and biosynthetic precursors of CoQ10 include, but are not limited to, those compounds that are formed between the chemical/biological conversion of tyrosine and acetyl-CoA to ubiquinol. Intermediates of the coenzyme biosynthesis pathway include tyrosine, acetyl-CoA, 3-hexaprenyl-4-hydroxybenzoate, 3-hexaprenyl-4,5-dihydroxybenzoate, 3-hexaprenyl-4-hydroxy-5-methoxybenzoate, 2-hexaprenyl-6-methoxy-1,4-benzoquinone, 2-hexaprenyl-3-methyl-6-methoxy-1,4-benzoquinone, 2-hexaprenyl-3-methyl-5-hydroxy-6-methoxy-1,4-benzoquinone, 3-Octaprenyl-4-hydroxybenzoate, 2-octaprenylphenol, 2-octaprenyl-6-metholxyphenol, 2-octaprenyl-3-methyl-6-methoxy-1,4-benzoquinone, 2-octaprenyl-3-methyl-5-hydroxy-6-methoxy-1,4-benzoquinone, 2-decaprenyl-3-methyl-5-hydroxy-6-methoxy-1,4-benzoquinone, 2-decaprenyl-3-methyl-6-methoxy-1,4-benzoquinone, 2-decaprenyl-6-methoxy-1,4-benzoquinone, 2-decaprenyl-6-methoxyphenol, 3-decaprenyl-4-hydroxy-5-methoxybenzoate, 3-decaprenyl-4,5-dihydroxybenzoate, 3-decaprenyl-4-hydroxybenzoate, 4-hydroxy phenylpyruvate, 4-hydroxyphenyllactate, 4-hydroxybenzoate, 4-hydroxycinnamate, and hexaprenydiphosphate

### III. Compositions

The present disclosure provides compositions containing CoQ10 for the treatment and prevention of cancer as specified in appended claim 1. In treating a patient exhibiting a cancer as specified in appended claim 1, a therapeutically effective amount of the CoQ10 compound is administered. A therapeutically effective dose refers to that amount of the compound which results in at least stable disease or a prolongation of survival in a patient.

Suitable routes of administration of the present compositions of the invention may include parenteral delivery, including, intravenous, intramuscular, subcutaneous, intramedullary injections, as well as intrathecal, direct intraventricular, intraperitoneal, intranasal, or intraocular injections, just to name a few. In one embodiment, the compositions provided herein may be administered by injecting directly to a tumor. In some embodiments, the formulations of the invention may be administered by intravenous injection or intravenous infusion. In some embodiments, the formulation is administered by continuous infusion. In one embodiment, the compositions of the invention are administered by intravenous injection. In one embodiment, the compositions of the invention are administered by intravenous infusion. Where the route of administration is, for example intravenous infusion, embodiments are provided herein where the IV infusion comprises the active agent CoQ10 at approximately a 40 mg/mL concentration. Where the composition is administered by IV infusion, it can be diluted in a pharmaceutically acceptable aqueous solution such as phosphate buffered saline or normal saline. In some embodiments, one or more routes of administration may be combined, such as, for example, intravenous and intratumoral, or intravenous and peroral, or intravenous and oral, or intravenous and topical, transdermal, or transmucosal.

The compositions described herein may be administered to a subject in any suitable formulation. These include, for example, liquid, semi-solid, and solid dosage forms, such as liquid solutions (*e.g*., injectable and infusible solutions), dispersions or suspensions, tablets, pills, powders, creams, lotions, liniments, ointments, or pastes, drops for administration to the eye, ear or nose, liposomes, and suppositories. The preferred form depends on the intended mode of administration and therapeutic application.

In certain embodiments, CoQ10 may be prepared with a carrier that will protect against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g*., Sustained and Controlled Release Drug Delivery Systems, J.R. Robinson, ed., Marcel Dekker, Inc., New York, 1978.

For example, CoQ10 can be formulated for parenteral delivery, *e.g*., for subcutaneous, intravenous, intramuscular, or intratumoral injection. The compositions may be administered in a single bolus, multiple injections, or by continuous infusion (for example, intravenously or by peritoneal dialysis). For parenteral administration, the compositions may be formulated in a sterilized pyrogen-free form.

Use of pharmaceutically acceptable carriers to formulate the compounds herein disclosed, for the practice of the present invention, into dosages suitable for systemic administration is within the scope of the present disclosure. With proper choice of carrier and suitable manufacturing practice, the compositions of the present disclosure, in particular, those formulated as solutions, may be administered parenterally, such as by intravenous injection.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices may be desirable. The data obtained from these cell culture assays and animal studies can be used in formulating a range of dosage for use in human. The dosage of such compounds may be within a range of circulating concentrations that include the ED50 with little or no toxicity. The dosage may vary within this range depending upon the dosage form employed and the route of administration utilized.

Pharmaceutical compositions for use according to the present invention include compositions wherein the active ingredients are contained in an effective amount to achieve its intended purpose. Determination of the effective amounts is well within the capability of those skilled in the art, especially in light of the detailed disclosure provided herein. In addition to the active ingredients, these pharmaceutical compositions may contain suitable pharmaceutically acceptable carriers including excipients and auxiliaries which facilitate processing of the active compounds into preparations which can be used pharmaceutically. The preparations formulated for intravenous administration may be in the form of solutions of colloidal dispersion.

Pharmaceutical compositions for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

### IV. Formulations

The active agent, CoQ10, can be delivered in any pharmaceutically acceptable carrier for the desired route of administration. As used herein, formulations including CoQ10 are formulated for any route of administration unless otherwise clearly indicated. In preferred embodiments, the formulations are for administration by injection, infusion, or topical administration. In certain embodiments, CoQ10 is not delivered orally.

Preferred therapeutic formulations for use in the methods of the invention comprise the active agent CoQ10 in a microparticle formation, e.g., for intravenous administration. Such intravenous formulations are provided, for example, in WO2011/112900 (Appln. No. PCT/US2011/028042), and an exemplary intravenous formulation as described in WO2011/112900 (Appln. No. PCT/US2011/028042) is used in the examples set forth below. Through high pressure homogenization, active agent CoQ10 particles are reduced to produce particles that are small enough to pass through a 200-nm sterilizing filter. Particles that are small enough to pass through a 200-nm sterilizing filter can be injected intravenously. These particles are much smaller than blood cells and therefore will not embolize capillaries. Red blood cells for example are 6-micron x 2-micron disks. The particles are dispersed to and are encased or surrounded by a stabilizing agent. While not wishing to be bound by any theory, it is believed that the stabilizing agents are attracted to the hydrophobic therapeutic agent such that the dispersed particles of the hydrophobic therapeutic agent are surrounded by the stabilizing agent forming a suspension or an emulsion. The dispersed particles in the suspension or emulsion comprises a stabilizing agent surface and a core consisting of the hydrophobic therapeutic agent, CoQ10, in a solid particulate form (suspension) or in an immiscible liquid form (emulsion). The dispersed particles can be entrenched in the lipophilic regions of a liposome.

Dispersed colloidal systems permit a high drug load in the formulation without the use of co-solvents. Additionally, high and relatively reproducible plasma levels are achieved without the dependence on endogenous low-density lipoprotein carriers. More importantly, the formulations allow sustained high drug levels in solid tumors due to the passive accumulation of the colloidal particles of the hydrophobic therapeutic agent.

A preferred intravenous formulation substantially comprises a continuous phase of water and dispersed solids (suspension) or dispersed immiscible liquid (emulsion). Dispersed colloidal systems, in which the particles are composed largely of the active agent (drug) itself, can often deliver more drug per unit volume than continuous solubilizing systems, if the system can be made adequately stable.

As the formulation medium, the aqueous solution may include Hank's solution, Ringer's solution, phosphate buffered saline (PBS), physiological saline buffer or other suitable salts or combinations to achieve the appropriate pH and osmolarity for parenterally delivered formulations. Aqueous solutions can be used to dilute the formulations for administration to the desired concentration. For example, aqueous solutions can be used to dilute a formulation for intravenous administration from a concentration of about 4% w/v to a lower concentration to facilitate administration of lower doses of CoQ10. The aqueous solution may contain substances which increase the viscosity of the solution, such as sodium carboxymethyl cellulose, sorbitol, or dextran.

The active agent CoQ10 is dispersed in the aqueous solution such that a colloidal dispersion is formed wherein the nano-dispersion particles of the hydrophobic therapeutic agent are covered or encased or encircled by the dispersion stabilizing agents to form nano-dispersions of the active agent CoQ10 particles. The nano-dispersed active agent CoQ10 particles have a core formed of the hydrophobic therapeutic agent that is surrounded by the stabilizing agent. Similarly, in certain aspects, the stabilizing agent is a phospholipid having both a hydrophilic and lipophilic portion. The phospholipids form liposomes or other nanoparticles upon homogenization. In certain aspects these liposomes are bi-layered unilamellar liposomes while in other embodiments the liposomes are bi-layered multi-lamellar liposomes. The dispersed active agent CoQ10 particles are dispersed in the lipophilic portion of the bi-layered structure of the liposome formed from the phospholipids. In certain other aspects the core of the liposome, like the core of the nano-dispersion of active agent CoQ10 particles, is formed of the hydrophobic therapeutic agent and the outer layer is formed of the bi-layered structure of the phospholipid. In certain embodiments the colloidal dispersions are treated by a lyophilization process whereby the nanoparticle dispersion is converted to a dry powder.

In some embodiments, the formulation for injection or infusion used is a 4% sterile aqueous colloidal dispersion containing CoQ10 in a nanosuspension as prepared in WO2011/112900. In certain embodiments, the formulation includes an aqueous solution; a hydrophobic active agent, CoQ10, dispersed to form a colloidal nano-dispersion of particles; and at least one of a dispersion stabilizing agent and an opsonization reducer; wherein the colloidal nano-dispersion of the active agent is dispersed into nano-dispersion particles having a mean size of less than 200-nm.

In certain embodiments, the dispersion stabilizing agent includes, but is not limited to, pegylated castor oil, Cremphor® EL, Cremophor® RH 40, Pegylated vitamin E, Vitamin E TPGS, and Dimyristoylphosphatidyl choline (DMPC).

In certain embodiments, the opsonization reducer is a poloxamer or a poloxamines.

In certain embodiments, the colloidal nano-dispersion is a suspension or an emulsion. Optionally, a colloidal nano-dispersion is in a crystalline form or a supercooled melt form.

In certain embodiments, the formulation for injection or infusion includes a lyoprotectant such as a nutritive sugar including, but not limited to, lactose, mannose, maltose, galactose, fructose, sorbose, raffinose, neuraminic acid, glucosamine, galactosamine, N-methylglucosamine, mannitol, sorbitol, arginine, glycine and sucrose, or any combination thereof.

In certain embodiments, the formulation for injection or infusion includes an aqueous solution; a hydrophobic active agent dispersed to form a colloidal nano-dispersion of particles; and at least one of a dispersion stabilizing agent and an opsonization reducer. The colloidal nano-dispersion of the active agent is dispersed into nano-dispersion particles having sizes of less than 200-nm. In some embodiments the dispersion stabilizing agent is selected from natural or semisynthetic phospholipids. For example, suitable stabilizing agents include polyethoxylated (a/k/a pegylated) castor oil (Cremophor® EL), polyethoxylated hydrogenated castor oil (Cremophor® RH 40), Tocopherol polyethylene glycol succinate (Pegylated vitamin E, Vitamin E TPGS), Sorbitan fatty acid esters (Spans®), Bile acids and bile-acid salts or Dimyristoylphosphatidyl choline (DMPC). In some embodiments the stabilizing agent is DMPC.

In certain embodiments the formulation is suitable for parenteral administration, including intravenous, intraperitoneal, orthotopical, intracranial, intramuscular, subcutaneous, intramedullary injections, as well as intrathecal, direct intraventricular, intranasal, or intraocular injections. In certain embodiments, the formulation contains CoQ10, dimyristoyl-phophatidylcholine, and poloxamer 188 in a ratio of 4:3:1.5 respectively that is designed to stabilize the nanosuspension of the particles. In some embodiments, the formulation includes a phosphate buffer saline solution which contains sodium phosphate dibasic, potassium phosphate monobasic, potassium chloride, sodium chloride and water for injection. In certain embodiments, the 4% sterile aqueous colloidal dispersion containing CoQ10 in a nanosuspension is diluted in the phosphate buffered saline solution provided, e.g., 1:1, 1:2, 1:3, 1:4. 1:5, 1:6, 1:7, 1:8. 1:9, 1:10, 1:11, 1:12, 1:13, 1:14. 1:15, 1:16, 1:17, 1:18. 1:19, 1:20, or other appropriate ratio bracketed by any two of the values.

In some embodiments, the formulation is a topical formulation. Topical formulations of CoQ10 compounds are provided, for example in WO2010/132507 (PCT Appln. No. PCT/US2010/034453), WO2008116135 (PCT Appln. No. PCT/US2008/116135), and WO2005/069916 (PCT Appln. PC/US2005/001581).

Formulations suitable for topical administration include liquid or semi-liquid preparations suitable for penetration through the skin, such as liniments, lotions, creams, ointments or pastes, and drops suitable for administration to the eye, ear, or nose. Drops according to the present disclosure may include sterile aqueous or oily solutions or suspensions and may be prepared by dissolving the active ingredient in a suitable aqueous solution of a bactericidal and/or fungicidal agent and/or any other suitable preservative, and in some embodiments including a surface active agent. The resulting solution may then be clarified and sterilized by filtration and transferred to the container by an aseptic technique. Examples of bactericidal and fungicidal agents suitable for inclusion in the drops are phenylmercuric nitrate or acetate (0.002%), benzalkonium chloride (0.01 %) and chlorhexidine acetate (0.01 %). Suitable solvents for the preparation of an oily solution include glycerol, diluted alcohol and propylene glycol.

Lotions according to the present disclosure include those suitable for application to the skin or eye. An eye lotion may include a sterile aqueous solution optionally containing a bactericide and may be prepared by methods similar to those for the preparation of drops. Lotions or liniments for application to the skin may also include an agent to hasten drying and to cool the skin, such as an alcohol, and/or a moisturizer such as glycerol or an oil such as castor oil or arachis oil.

Creams, ointments or pastes useful in the methods of the invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy basis. The basis may include hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol or macrogels. The formulation may incorporate any suitable surface active agent such as an anionic, cationic or non-ionic surface active such as sorbitan esters or polyoxyethylene derivatives thereof Suspending agents such as natural gums, cellulose derivatives or inorganic materials such as silicaceous silicas, and other ingredients such as lanolin, may also be included.

In some embodiments, the remaining component of a topical delivery vehicle may be water or a water phase, in embodiments purified, e.g. deionized, water, glycerine, propylene glycol, ethoxydiglycol, phenoxyethanol, and cross linked acrylic acid polymers. Such delivery vehicle compositions may contain water or a water phase in an amount of from about 50 to about 95 percent, based on the total weight of the composition. The specific amount of water present is not critical, however, being adjustable to obtain the desired viscosity (usually about 50 cps to about 10,000 cps) and/or concentration of the other components. The topical delivery vehicle may have a viscosity of at least about 30 centipoises.

Topical formulations can also include an oil phase including, for example, oil phase which, in turn, may include emollients, fatty alcohols, emulsifiers, combinations thereof, and the like. For example, an oil phase could include emollients such as C12-15 alkyl benzoates (commercially available as FINSOLV™ TN from Finetex Inc. (Edison, N.J.)), capric-caprylic triglycerides (commercially available from Huls as MIGLYOL™ 812), and the like. Other suitable emollients which may be utilized include vegetable derived oils (corn oil, safflower oil, olive oil, macadamian nut oil, etc.); various synthetic esters, including caprates, linoleates, dilinoleates, isostearates, fumarates, sebacates, lactates, citrates, stearates, palmitates, and the like; synthetic medium chain triglycerides, silicone oils or polymers; fatty alcohols such as cetyl alcohol, stearyl alcohol, cetearyl alcohol, lauryl alcohol, combinations thereof, and the like; and emulsifiers including glyceryl stearate, PEG-100 stearate, Glyceryl Stearate, Glyceryl Stearate SE, neutralized or partially neutralized fatty acids, including stearic, palmitic, oleic, and the like; vegetable oil extracts containing fatty acids, Ceteareth®-20, Ceteth®-20, PEG-150 Stearate, PEG-8 Laurate, PEG-8 Oleate, PEG-8 Stearate, PEG-20 Stearate, PEG-40 Stearate, PEG-150 Distearate, PEG-8 Distearate, combinations thereof, and the like; or other non-polar cosmetic or pharmaceutically acceptable materials used for skin emolliency within the purview of those skilled in the art, combinations thereof, and the like.

Topical formulations can also include a liposomal concentrate including, for example, a phospholipid such as lecithin, lysolecithin, phosphatidylcholine, phosphatidylethanolamine, phosphatidylinositol, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, and combinations thereof, at least one lipophilic bioactive agent, and at least one solubilizer. The liposomal concentrate may be in combination with at least one pharmaceutically acceptable carrier possessing at least one permeation enhancer in an amount from about 0.5% by weight to about 20% by weight of the composition. The phospholipid may present in the composition in an amount from about 2% to about 20% by weight of the composition and the bioactive agent may be present in an amount from about 0.5% to about 20% by weight of the composition.

Transdermal skin penetration enhancers can also be used to facilitate delivery of CoQ10. Illustrative are sulfoxides such as ethoxydiglycol, 1,3-butylene glycol, isopentyl diol, 1,2-pentane diol, propylene glycol, 2-methyl propan-2-ol, propan-2-ol, ethyl-2-hydroxypropanoate, hexan-2,5-diol, di(2-hydroxypropyl)ether, pentan-2,4-diol, acetone, polyoxyethylene(2)methyl ether, 2-hydroxypropionic acid, 2-hydroxyoctanoic acid, propan-1-ol, 1,4 dioxane, tetrahydrofuran, butan-1,4-diol, propylene glycol dipelargonate, polyoxypropylene 15 stearyl ether, octyl alcohol, polyoxyethylene ester of oleyl alcohol, oleyl alcohol, lauryl alcohol, dioctyl adipate, dicapryl adipate, diisopropyl adipate, diisopropyl sebacate, dibutyl sebacate, diethyl sebacate, dimethyl sebacate, dioctyl sebacate, dibuyl suberate, dioctyl azelate, dibenzyl sebacate, dibutyl phthalate, dibutyl azelate, ethyl myristate, dimethyl azelate, butyl myristate, dibutyl succinate, didecyl phthalate, decyl oleate, ethyl caproate, ethyl salicylate, isopropyl palmitate, ethyl laurate, 2-ethyl-hexylpelargonate, isopropyl isostearate, butyl laurate, benzyl benzoate, butyl benzoate, hexyl laurate, ethyl caprate, ethyl caprylate, butyl stearate, benzyl salicylate, 2-hyroxyoctanoic acid, dimethyl sulphoxide, methyl sufonyl methane, n,n-dimethyl acetamide, n,n-dimethyl formamide, 2-pyrrolidone, 1-methyl-2-pyrrolidone, 5-methyl-2-pyrrolidone, 1,5-dimethyl-2-pyrrolidone, 1-ethyl-2-pyrrolidone, phosphine oxides, sugar esters, tetrahydrofurfural alcohol, urea, diethyl-m-toluamide, 1-dodecylazacyloheptan-2-one, and combinations thereof.

Solubilizers, particularly for topical administration can include, but are not limited to, polyoxyalkylene dextrans, fatty acid esters of saccharose, fatty alcohol ethers of oligoglucosides, fatty acid esters of glycerol, fatty acid esters of polyoxyethylenes, polyethoxylated fatty acid esters of sorbitan, fatty acid esters of poly(ethylene oxide), fatty alcohol ethers of poly(ethylene oxide), alkylphenol ethers of poly(ethylene oxide), polyoxyethylene-polyoxypropylene block copolymers, ethoxylated oils, and combinations thereof

Topical formulations can include emollients, including, but not limited to, C12-15 alkyl benzoates, capric-caprylic triglycerides, vegetable derived oils, caprates, linoleates, dilinoleates, isostearates, fumarates, sebacates, lactates, citrates, stearates, palmitates, synthetic medium chain triglycerides, silicone oils, polymers and combinations thereof; the fatty alcohol is selected from the group consisting of cetyl alcohol, stearyl alcohol, cetearyl alcohol, lauryl alcohol and combinations thereof; and the emulsifier is selected from the group consisting of glyceryl stearate, polyethylene glycol 100 stearate, neutralized fatty acids, partially neutralized fatty acids, polyethylene glycol 150 stearate, polyethylene glycol 8 laurate, polyethylene glycol oleate, polyethylene glycol 8 stearate, polyethylene glycol 20 stearate, polyethylene glycol 40 stearate, polyethylene glycol 150 distearate, polyethylene glycol 8 distearate, and combinations thereof

Topical formulations can include a neutralization phase comprising one or more of water, amines, sodium lactate, and lactic acid.

The water phase can further optionally include one or more of water phase comprises the permeation enhancer optionally in combination with a viscosity modifier selected from the group consisting of cross linked acrylic acid polymers, pullulan, mannan, scleroglucans, polyvinylpyrrolidone, polyvinyl alcohol, guar gum, hydroxypropyl guar gum, xanthan gum, acacia gum, arabia gum, tragacanth, galactan, carob gum, karaya gum, locust bean gum, carrageenin, pectin, amylopectin, agar, quince seed, rice starch, corn starch, potato starch, wheat starch, algae extract, dextran, succinoglucan, carboxymethyl starch, methylhydroxypropyl starch, sodium alginate, alginic acid propylene glycol esters, sodium polyacrylate, polyethylacrylate, polyacrylamide, polyethyleneimine, bentonite, aluminum magnesium silicate, laponite, hectonite, and anhydrous silicic acid.

Topical formulations can also include a pigment such as titanium dioxide.

In an embodiment, a topical formulation for use in the methods of the invention includes an oil phase comprising C12-15 alkyl benzoates or capric/caprylic triglyceride, cetyl alcohol, stearyl alcohol, glyceryl stearate, and polyethylene glycol 100 stearate, in an amount of from about 5% to about 20% by weight of the composition; a water phase comprising glycerin, propylene glycol, ethoxydiglycol, phenoxyethanol, water, and a crosslinked acrylic acid polymer, in an amount of from about 60 to about 80% by weight of the composition; a neutralization phase comprising water, triethanolamine, sodium lactate, and lactic acid, in an amount of from about 0.1% to about 15% by weight of the composition; a pigment comprising titanium dioxide in an amount of from about 0.2% to about 2% by weight of the composition; and a liposomal concentrate comprising a polyethoxylated fatty acid ester of sorbitan, coenzyme Q10, a phosphatidylcholine lecithin, phenoxyethanol, propylene glycol, and water, in an amount of from about 0.1% to about 30% by weight of the composition, wherein the propylene glycol and ethoxydiglycol are present in a combined amount of from 3% by weight to about 15% by weight of the composition and the coenzyme Q10 is present in an amount of from about 0.75% by weight to about 10% by weight of the composition. Other formulations for use in the methods of the invention are provided, for example, in WO2008/116135 (PCT Application No. PCT/US08/57786), and in WO2010/132507 (PCT/US2010/034453).

In one embodiment, a topical formulation for use in the methods of the invention is a 3% CoQ10 cream as described in US 2011/0027247. In one embodiment, the 3% CoQ10 comprises:
(1) a phase A having C12-15 alkyl benzoate or capric/caprylic triglyceride at about 4.0% w/w of the composition, cetyl alcohol at about 2.00% w/w of the composition, stearyl alcohol at about 1.5% w/w, glyceryl stearate and PEG-100 at about 4.5% w/w;
(2) a phase B having glycerin at about 2.00% w/w, propylene glycol at about 1.5% w/w, ethoxydiglycol at about 5.0% w/w, phenoxyethanol at about 0.475% w/w, a carbomer dispersion at about 40% w/w, purified water at about 16.7% w/w;
(3) a phase C having triethanolamine at about 1.3% w/w, lactic acid at about 0.5% w/w, sodium lactate solution at about 2.0% w/w, water at about 2.5% w/w;
(4) a phase D having titanium dioxide at about 1.0% w/w; and
(5) a phase E having CoQ10 21% concentrate at about 15.0% w/w.

A CoQ10 21% concentrate composition (phase E in above 3% cream) can be prepared by combining phases A and B as described below. Phase A includes Ubidecarenone USP (CoQ10) at 21 %w/w and polysorbate 80 NF at 25 %w/w. Phase B includes propylene glycol USP at 10.00 %w/w, phenoxyethanol NF at 0.50 %w/w, lecithin NF (PHOSPHOLIPON 85G) at 8.00 %w/w and purified water USP at 35.50 %w/w. All weight percentages are relative to the weight of the entire CoQ10 21% concentrate composition. The percentages and further details are listed in the following table.

**Table 1**

| **Phase** | **Trade Name** | **INCI Name** | **Percent** |
|---|---|---|---|
| **A** | RITABATE 80 | POLYSORBATE 80 | 25.000 |
| **A** | UBIDECARENONE | UBIQUINONE | 21.000 |
| **B** | PURIFIED WATER | WATER | 35.500 |
| **B** | PROPYLENE GLYCOL | PROPYLENE GLYCOL | 10.000 |
| **B** | PHENOXYETHANOL | PHENOXYETHANOL | 0.500 |
| **B** | PHOSPHOLIPON 85G | LECITHIN | 8.000 |
| **Totals** | | | 100.000 |

The phenoxyethanol and propylene glycol are placed in a suitable container and mixed until clear. The required amount of water is added to a second container (Mix Tank 1). Mix Tank 1 is heated to between 45 and 55 °C while being mixed. The phenoxyethanol/propylene glycol solution is added to the water and mixed until it was clear and uniform. When the contents of the water phase in Mix Tank 1 are within the range of 45 to 55 °C, Phospholipon G is added with low to moderate mixing. While avoiding any foaming, the contents of Mix Tank 1 is mixed until the Phospholipon 85G was uniformly dispersed. The polysorbate 89 is added to a suitable container (Mix Tank 2) and heated to between 50 and 60 °C. The Ubidecarenone is then added to Mix Tank 2. While maintaining the temperature at between 50 and 60 °C Mix Tank 2 is mixed until all the Ubidecarenone is dissolved. After all the Ubidecarenone has been dissolved, the water phase is slowly transferred to Mix Tank 2. When all materials have been combined, the contents are homogenized until dispersion is smooth and uniform. While being careful not to overheat, the temperature is maintained at between 50 and 60 °C. The homogenization is then stopped and the contents of Mix Tank 2 are transferred to a suitable container for storage.

In some embodiments, a formulation for any route of administration for use in the invention may include from about 0.001% to about 20% (w/w) of CoQ10, more preferably between about 0.01% and about 15% and even more preferably between about 0.1% to about 10% (w/w) of CoQ10. In certain embodiments, a formulation for any route of administration for use in the invention may include from about 1% to about 10% (w/w) of CoQ10. In certain embodiments, a formulation for any route of administration for use in the invention may include from about 2% to about 8% (w/w) of CoQ10. In certain embodiments, a formulation for any route of administration for use in the invention may include from about 2% to about 7% (w/w) of CoQ10. In certain embodiments, a formulation for any route of administration for use in the invention may include from about 3% to about 6% (w/w) of CoQ10. In certain embodiments, a formulation for any route of administration for use in the invention may include from about 3% to about 5% (w/w) of CoQ10. In certain embodiments, a formulation for any route of administration for use in the invention may include from about 3.5% to about 4.5% (w/w) of CoQ10. In certain embodiments, a formulation for any route of administration for use in the invention may include from about 3.5% to about 5% (w/w) of CoQ10. In one embodiment a formulation includes about 4% (w/w) of CoQ10. In one embodiment a formulation includes about 8% (w/w) of CoQ10. In various embodiments, the formulation includes about 0.1%, 0.2%, 0.3%, 0.4%, 0.5%, 1%, 2%, 3%, 4%, 5%, 6%, 7%, 8%, 9%, 10%, 11%, 12%, 13%, 14%, 15%, 16%, 17%, 18%, 19% or 20% (w/w) of CoQ10, or any range bracketed by any two values recited. In certain embodiments, the formulations can be prepared as a percent weight to volume rather than a percent weight to weight. Depending on the formulation, the concentration of CoQ10 may be the same, or about the same in the w/w and the w/v percent formulations. CoQ10 can be obtained from Kaneka Q10 as Kaneka Q10 (USP UBIDECARENONE) in powdered form (Pasadena, Texas, USA). CoQ10 used in the methods exemplified herein have the following characteristics: residual solvents meet USP 467 requirement; water content is less than 0.0%, less than 0.05% or less than 0.2%; residue on ignition is 0.0%, less than 0.05%, or less than 0.2% less than; heavy metal content is less than 0.002%, or less than 0.001%; purity of between 98-100% or 99.9%, or 99.5%.

In certain embodiments, the concentration of CoQ10 in the formulation is 1 mg/mL to 150 mg/mL. In one embodiment, the concentration of CoQ10 in the formulation is 5 mg/mL to 125 mg/mL. In one embodiment, the concentration of CoQ10 in the formulation is 10 mg/mL to 100 mg/mL. In one embodiment, the concentration of CoQ10 in the formulation is 20 mg/mL to 90 mg/mL. In one embodiment, the concentration of CoQ10 is 30 mg/mL to 80 mg/mL. In one embodiment, the concentration of CoQ10 is 30 mg/mL to 70 mg/mL. In one embodiment, the concentration of CoQ10 is 30 mg/mL to 60 mg/mL. In one embodiment, the concentration of CoQ10 is 30 mg/mL to 50 mg/mL. In one embodiment, the concentration of CoQ10 is 35 mg/mL to 45 mg/mL. It should be understood that additional ranges having any one of the foregoing values as the upper or lower limits are also intended to be part of this invention, e.g., 10 mg/mL to 50 mg/mL, or 20 mg/mL to 60 mg/mL.

In certain embodiments, the concentration of CoQ10 in the formulation is about 10, 15, 20, 25, 30, 31, 32, 33, 34, 35, 36, 37, 38, 39, 40, 41, 42, 43, 44, 45, 46, 47, 48, 49, 50, 55, 60, 65, 70, 75, 80, 85, 90 or 95 mg/mL. In one embodiment, the concentration of CoQ10 in the formulation is about 50 mg/mL. In one embodiment, the concentration of CoQ10 in the formulation is about 60 mg/mL. In one embodiment, the concentration of CoQ10 in the formulation is about 30 mg/mL. In a preferred embodiment, the concentration of CoQ10 in the formulation is about 40 mg/mL. It should be understood that ranges having any one of these values as the upper or lower limits are also intended to be part of this invention, e.g. between 37 mg/mL and 47 mg/mL, or between 31 mg/mL and 49 mg/mL.

It is understood that formulations can similarly be prepared containing CoQ10 precursors, metabolites, and related compounds.

### V. Treatment of Cancer

Formulations of the present disclosure may be utilized for the treatment of solid tumors wherein the subject has failed at least one prior chemotherapeutic regimen. Accordingly, the present invention provides compositions for use in methods of treating cancer in a subject, wherein the subject has failed at least one prior chemotherapeutic regimen for the cancer, comprising administering the formulations of the invention to the subject in an amount sufficient to treat the cancer, thereby treating cancer, wherein the cancer is as specified in appended claim 1. The formulations of the invention may also be utilized for inhibiting tumor cell growth in a subject wherein the subject has failed at least one prior chemotherapeutic regimen, wherein the cancer is as specified in appended claim 1. Accordingly, the invention further provides compositions for use in methods of inhibiting tumor cell growth in a subject, wherein the subject has failed at least one prior chemotherapeutic regimen, comprising administering the formulations of the invention to the subject, such that tumor cell growth is inhibited, wherein the tumor is associated with cancer is as specified in appended claim 1. In a preferred embodiment, inhibiting tumor growth includes achieving at least stable disease of the primary lesion by RECIST 1.1 criteria. In certain embodiments, the subject is a human subject.

Such formulations may include the hydrophobic therapeutic agent, CoQ10, in a pharmaceutically acceptable carrier. In some embodiments, such a formulation may include from about 0.001% to about 20% (w/w) of CoQ10, more preferably between about 0.01% and about 15% and even more preferably between about 0.1% to about 10% (w/w) of CoQ10. In one embodiment a formulation includes about 4% (w/w) of CoQ10. In one embodiment a formulation includes about 8% (w/w) of CoQ10. In various embodiments, the formulation includes about 0.1%, 0.2%. 0.3%, 0.4%. 0.5%, 0.6%, 0.7%, 0.8%. 0.9%, 1%, 2%, 3%, 4%, 5%, 6%, 7%, 8%, 9%, 10%, 11%, 12%, 13%, 14%, 15%, 16%, 17%, 18%, 19% or 20% (w/w) of CoQ10, or any range bracketed by those values. In certain embodiments, the formulations can be prepared as a percent weight to volume rather than a percent weight to weight. Depending on the formulation, the concentration of CoQ10 may be the same, or about the same in the w/w and the w/v percent formulations. As also noted herein, compositions of the present disclosure may be in a liquid form, capable of introduction into a subject by any means or route of administration within the purview of those skilled in the art. For example, compositions may be administered by routes of administration including, but not limited to, intravenous, intratumoral, combinations thereof, and the like.

In certain embodiments of the invention, compositions for use are provided for treating or preventing cancer in a human by intravenously administering a CoQ10 formulation to the human such that treatment or prevention occurs, wherein the human is administered a dose of the formulation such that, preferably, CoQ10 is administered in the range of about 0.5 mg/kg/dose to about 10,000 mg/kg/dose, about 5 mg/kg/dose to about 5,000 mg/kg/dose, about 10 mg/kg/dose to about 3,000 mg/kg/dose. In one embodiment, the formulation is administered such that, preferably, CoQ10 is administered in the range of about 10 mg/kg/dose to about 1,400 mg/kg/dose. In one embodiment, the formulation is administered such that, preferably, CoQ10 is administered in the range of about 10 mg/kg/dose to about 650 mg/kg/dose. In one embodiment, the formulation is administered such that, preferably, CoQ10 is administered in the range of about 10 mg/kg/dose to about 200 mg/kg/dose. In various embodiments, the formulation is administered such that, preferably, CoQ10 is administered at a dose of about 2mg/kg/dose, 5 mg/kg/dose, 10 mg/kg/dose, 15 mg/kg/dose, 20 mg/kg/dose, 25 mg/kg/dose, 30 mg/kg/dose, 35 mg/kg/dose, 40 mg/kg/dose, 45 mg/kg/dose, 50 mg/kg/dose, 55 mg/kg/dose, 56 mg/kg/dose, 57 mg/kg/dose, 58 mg/kg/dose, 59 mg/kg/dose, 60 mg/kg/dose, 65 mg/kg/dose, 70 mg/kg/dose, 75 mg/kg/dose, 76 mg/kg/dose, 77 mg/kg/dose, 78 mg/kg/dose, 79 mg/kg/dose, 80 mg/kg/dose, 85 mg/kg/dose, 90 mg/kg/dose, 95 mg/kg/dose, 100 mg/kg/dose, 101 mg/kg/dose, 102 mg/kg/dose, 103 mg/kg/dose, 104 mg/kg/dose, 105 mg/kg/dose, 106 mg/kg/dose, 107 mg/kg/dose, 108 mg/kg/dose, 109 mg/kg/dose, 110 mg/kg/dose, 120 mg/kg/dose, 130 mg/kg/dose, 140 mg/kg/dose, 150 mg/kg/dose, 160 mg/kg/dose, 170 mg/kg/dose, 180 mg/kg/dose, 190 mg/kg/dose, or 200 mg/kg/dose. In various embodiments, the formulation is administered such that, preferably, CoQ10 is administered at a dose of at least 2mg/kg/dose, 5 mg/kg/dose, 10 mg/kg/dose, 15 mg/kg/dose, 20 mg/kg/dose, 25 mg/kg/dose, 30 mg/kg/dose, 35 mg/kg/dose, 40 mg/kg/dose, 45 mg/kg/dose, 50 mg/kg/dose, 55 mg/kg/dose, 56 mg/kg/dose, 57 mg/kg/dose, 58 mg/kg/dose, 59 mg/kg/dose, 60 mg/kg/dose, 65 mg/kg/dose, 70 mg/kg/dose, 75 mg/kg/dose, 76 mg/kg/dose, 77 mg/kg/dose, 78 mg/kg/dose, 79 mg/kg/dose, 80 mg/kg/dose, 85 mg/kg/dose, 90 mg/kg/dose, 95 mg/kg/dose, 100 mg/kg/dose, 101 mg/kg/dose, 102 mg/kg/dose, 103 mg/kg/dose, 104 mg/kg/dose, 105 mg/kg/dose, 106 mg/kg/dose, 107 mg/kg/dose, 108 mg/kg/dose, 109 mg/kg/dose, 110 mg/kg/dose, 120 mg/kg/dose, 130 mg/kg/dose, 140 mg/kg/dose, 150 mg/kg/dose, 160 mg/kg/dose, 170 mg/kg/dose, 180 mg/kg/dose, 190 mg/kg/dose, or 200 mg/kg/dose, wherein the dose does not result in any limiting toxicities. It should be understood that ranges having any one of these values as the upper or lower limits are also intended to be part of this invention, e.g., about 50 mg/kg/dose to about 200 mg/kg/dose, or about 650 mg/kg/dose to about 1400 mg/kg/dose, or about 55 mg/kg/dose to about 110 mg/kg/dose. In one embodiment the administered dose is at least about 1 mg/kg/dose, at least about 5 mg/kg/dose, at least about 10 mg/kg/dose, at least about 12.5 mg/kg/dose, at least about 20 mg/kg/dose, at least about 25 mg/kg/dose, at least about 30 mg/kg/dose, at least about 35 mg/kg/dose, at least about 40 mg/kg/dose, at least about 45 mg/kg/dose, at least about 50 mg/kg/dose, at least about 55 mg/kg/dose, at least about 60 mg/kg/dose, at least about 75 mg/kg/dose, at least about 100 mg/kg/dose, at least about 125 mg/kg/dose, at least about 150 mg/kg/dose, at least about 175 mg/kg/dose, at least about 200 mg/kg/dose, at least about 300 mg/kg/dose, or at least about 400 mg/kg/dose. In certain embodiments, the administered dose is no more than about 20 mg/kg/dose, about 25 mg/kg/dose, about 30 mg/kg/dose, about 35 mg/kg/dose, about 40 mg/kg/dose, about 45 mg/kg/dose, about 50 mg/kg/dose, about 55 mg/kg/dose, about 60 mg/kg/dose, about 75 mg/kg/dose, about 100 mg/kg/dose, about 125 mg/kg/dose, about 150 mg/kg/dose, about 175 mg/kg/dose, about 200 mg/kg/dose, about 300 mg/kg/dose, about 400 mg/kg/dose, about 500 mg/kg/dose, about 600 mg/kg/dose, about 700 mg/kg/dose, about 800 mg/kg/dose, about 900 mg/kg/dose, about 1000 mg/kg/dose, about 1100 mg/kg/dose, about 1200 mg/kg/dose, or about 1300 mg/kg/dose. It is understood that any of the lower limit values and upper limit values can be combined to create a range. In certain embodiments, the administered dose is at least 75 mg/kg/dose or 100 mg/kg/dose or the rat equivalent to about, at least, 12.2 or 16.2 mg/kg/day in humans, or at least 85 mg/kg over a week period, or at least 113 mg/kg over a week period.

In one embodiment, the CoQ10 formulation is administered one time per week. In one embodiment, the CoQ10 formulation is administered two times per week. In one embodiment, the CoQ10 formulation is administered 3 times per week. In one embodiment, the CoQ10 formulation is administered four times per week. In another embodiment, the CoQ10 formulation is administered 5 times per week. In one embodiment, the CoQ10 formulation, is administered once per day. In some embodiments, where the formulation is an IV formulation administered by infusion, the dosage is administered by infusion over about 1 hour, over about 2 hours, over about 3 hours, over about 4 hours, or longer. In one embodiment, the IV formulation is administered by infusion over about 4 hours, e.g., about 3.5 hours to about 4.5 hours. In certain embodiments, the formulation is administered over 4 or more hours. In certain embodiments, the formulation is administered over 8 or more hours. In certain embodiments, the formulation is administered over 12 hours or more. In certain embodiments, the formulation is administered over 18 or more hours. In certain embodiments, the formulation is administered over 24 or more hours. In certain embodiments, the formulation is administered over about 24 hours.

In certain embodiments, the CoQ10 formulation can be administered in one or more cycles. For example, the CoQ10 formulation can be administered for 2, 3, 4, 5, 6, 7, 8, or more weeks consecutively, and then not administered for a period of 1, 2, 3, 4, or more weeks, providing a cycle of administration. In certain embodiments, the cycles are administered without a pause between cycles. In certain embodiments, at the end of one or more cycles, the patient is assessed to determine treatment efficacy, toxicity, and assess if the treatment should be continued, modified, or ended. The number of cycles of administration depends, for example, on the response of the subject, the severity of disease, other therapeutic interventions used on the subject, or any adverse response of the subject. In certain embodiments, the CoQ10 formulation is administered as long as the subject is exhibiting at least a stable response to treatment with no serious adverse events, e.g., dose limiting toxicities, grade IV toxicities, or persistent grade III toxicities that cannot be mitigated by the use of other interventions.

In another embodiment, the CoQ10 formulation is administered in the form of a CoQ10 IV formulation at a dosage of between about 10 mg/kg/dose and about 10,000 mg/kg/dose of CoQ10, about 20 mg/kg/dose to about 5000 mg/kg/dose, about 50 mg/kg/dose to about 3000 mg/kg/dose, about 100 mg/kg/dose to about 2000 mg/kg/dose, about 200 mg/kg/dose to about 1000 mg/kg/dose, about 300 mg/kg/dose to about 500 mg/kg/dose, or about 55 mg/kg/dose to about 110 mg/kg/dose wherein the CoQ10 formulation comprises between about 1% and 10% of CoQ10 (w/v). In one embodiment, the CoQ10 formulation comprises about 4% of CoQ10 (w/v). In one embodiment, the CoQ10 IV formulation comprises about 8% of CoQ10 (w/v). In other embodiments, the CoQ10 IV formulation comprises about 0.1%, 0.2%. 0.3%, 0.4%. 0.5%, 0.6%, 0.7%, 0.8%. 0.9%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% of CoQ10 (w/v). It should be understood that ranges having any one of these values as the upper or lower limits are also intended to be part of this invention.

In the treatment of cancer, the formulations may be in a pharmaceutically acceptable carrier that may be administered in a therapeutically effective amount to a subject as either a mono-therapy, in combination with at least one other anticancer agent, e.g., chemotherapeutic agent, for a given indication, in combination with radiotherapy, following surgical intervention to radically remove a tumor, in combination with other alternative and/or complementary acceptable treatments for cancer, and the like.

In the present invention, the formulation is for use in treating a cancer as specified in appended claim 1 in a subject wherein the subject has failed at least one chemotherapeutic regimen.

In certain embodiments, the effect CoQ10 may have on cancer cells may depend, in part, on the various states of metabolic and oxidative flux exhibited by the cancer cells. CoQ10 may be utilized to interrupt and/or interfere with the conversion of an oncogenic cell's dependency of glycolysis and increased lactate utility. As it relates to a cancer state, this interference with the glycolytic and oxidative flux of the tumor microenvironment may influence apoptosis and angiogenesis in a manner which reduces the viability or proliferative capacity of a cancer cell. In some embodiments, the interaction of CoQ10 with glycolytic and oxidative flux factors may enhance the ability of CoQ10 to exert its restorative apoptotic effect in cancer.

Administration of CoQ10 as described herein, reduces tumor size, inhibits tumor growth and/or prolongs the survival time of a tumor-bearing subject who has failed at least one prior chemotherapeutic regimen as compared to an appropriate control. Accordingly, this invention also relates to compositions for use in a method of treating tumors in a human or other animal who has failed at least one prior chemotherapeutic regimen by administering to such human or animal an effective, non-toxic amount of the CoQ10 composition, for example, by administering an effective dose by IV administration. Or, for example, by administering an effective dose by topical administration. One skilled in the art would be able, by routine experimentation with the guidance provided herein, to determine what an effective, non-toxic amount of CoQ10 would be for the purpose of treating malignancies in a subject who has failed at least one prior chemotherapeutic regimen. For example, a therapeutically active amount of CoQ10 may vary according to factors such as the disease stage (e.g., stage I versus stage IV), age, sex, medical complications (e.g., immunosuppressed conditions or diseases) and weight of the subject, and the ability of the CoQ10 to elicit a desired response in the subject. The dosage regimen may be adjusted to provide the optimum therapeutic response. For example, several divided doses may be administered daily, the dose may be administered by continuous infusion, or the dose may be proportionally reduced as indicated by the exigencies of the therapeutic situation.

In certain embodiments of the invention, the compositions of the invention are for use in methods that further include a treatment regimen which includes any one of or a combination of surgery, radiation, chemotherapy, e.g., hormone therapy, antibody therapy, therapy with growth factors, cytokines, and anti-angiogenic therapy.

Cancers for treatment using the compositions of the invention are colon and rectal cancer, pancreatic cancer, liver cancer, oral cancers, uterine cancer and myxoid liposarcoma.

As used herein, the terms "cancer," "neoplasm," and "tumor," are used interchangeably and in either the singular or plural form, refer to cells that have undergone a malignant transformation that makes them pathological to the host organism. Primary cancer cells (that is, cells obtained from near the site of malignant transformation) can be readily distinguished from non-cancerous cells by well-established techniques, particularly histological examination. The definition of a cancer cell, as used herein, includes not only a primary cancer cell, but any cell derived from a cancer cell ancestor. This includes metastasized cancer cells, and in vitro cultures and cell lines derived from cancer cells. When referring to a type of cancer that normally manifests as a solid tumor, a "clinically detectable" tumor is one that is detectable on the basis of tumor mass; e.g., by procedures such as CAT scan, MR imaging, X-ray, ultrasound or palpation, and/or which is detectable because of the expression of one or more cancer-specific antigens in a sample obtainable from a patient.

The term "sarcoma" generally refers to a tumor which is made up of a substance like the embryonic connective tissue and is generally composed of closely packed cells embedded in a fibrillar or homogeneous substance.

The term "melanoma" is taken to mean a tumor arising from the melanocytic system of the skin and other organs. Melanomas include but are not limited to, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

The term "carcinoma" refers to a malignant new growth made up of epithelial cells tending to infiltrate the surrounding tissues and give rise to metastases. Carcinomas include but are not limited to, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypemephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, and carcinoma villosum.

Additional cancers include, for example, Hodgkin's disease, Non-Hodgkin's lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, cervical cancer, endometrial cancer, adrenal cortical cancer, prostate cancer, pancreatic cancer, uterine sarcoma, myxoid liposarcoma, leiomyosarcoma, chondrosarcoma, osteosarcoma, colon adenocarcinoma of colon, cervical squamous cell carcinoma, tonsil squamous cell carcinoma, papillary thyroid cancer, adenoid cystic cancer, synovial cell sarcoma, malignant fibrous histiocytoma, desmoplastic sarcoma, hepatocellular carcinoma, spindle cell sarcoma, cholangiocarcinoma, and triple negative breast cancer.

### VI. Treatment of Cancer After Failure of a Chemotherapeutic Regimen

The compositions of the invention and methods provided herein are for the treatment of cancer in a subject wherein the subject has previously failed at least one prior (e.g., 1, 2, 3, 4, 5, 6, 7, 8, 9, 10, or more) chemotherapeutic regimen for the cancer. A subject who has failed a chemotherapeutic regimen does not achieve at least stable disease or loses stable disease as defined by RECIST 1.1 criteria after a short period in at least one target lesion during or after the treatment with the chemotherapeutic regimen. In certain embodiments, a short period is less than 6 months. In certain embodiments, a short period is less than 5 months. In certain embodiments, a short period is less than 4 months. In certain embodiments, a short period is less than 3 months. In certain embodiments, a short period is less than 2 months. In certain embodiments, a short period is less than 1 month. In certain embodiments, a short period is less than 2 weeks. A subject who has failed a chemotherapeutic regimen includes a subject who has progressive disease during treatment or shortly after the end of treatment with a chemotherapeutic agent. In a preferred embodiment, the subject who has failed a chemotherapeutic regimen still has, or is suspected of having, the primary tumor. It is understood that it may not be possible or desirable to specifically identify the primary tumor, particularly when a subject presents with metastatic disease. In a preferred embodiment, the subject who has failed a chemotherapeutic regimen still has a tumor at the site of the primary tumor, i.e., in the organ in which the primary tumor arose. In certain embodiments, the primary tumor is a solid tumor.

A subject who has failed a chemotherapy has not been cured of the cancer being treated or according to a clinical definition, e.g., achieving complete remission in target or non-target lesions per the RECIST 1.1 criteria for an extended period after the conclusion of treatment of the cancer, e.g., for at least one year, at least 5 years, at least 10 years. For example, a subject treated for a pediatric cancer who is cured, i.e., who has achieved complete remission, has a greater chance of suffering from a distinct cancer later in life. A subject successfully treated for a cancer (i.e., a subject who achieves clinical remission for a sufficient time to be considered cured, e.g., at least 5 years of clinical remission) who later develops a second cancer is not considered to have failed the first chemotherapeutic regimen.

A subject who has failed a chemotherapeutic regimen may have also undergone other treatments for the cancer in conjunction with chemotherapy including surgery for tumor resection and/ or radiation therapy. The subject may have undergone bone marrow transplant or other procedures. It is understood that failure of a chemotherapeutic regimen may be due, at least in part, to a failure of one or more interventions other than the chemotherapy.

Failure of a chemotherapeutic regimen can result from the subject suffering from a dose limiting toxicity, e.g., a grade IV toxicity or a lower grade toxicity that cannot be tolerated by the subject or resolved with other interventions, e.g., anti-nausea agents, stimulators of red blood cell production, agents to normalize clotting, agents to reduce immune/allergic response, etc., depending on the specific toxicity. It is understood that such dose limiting toxicities can result in a shortened or incomplete chemotherapeutic regimen being administered to the subject, resulting in reduced efficacy of the agent.

### VII. Combination Therapies

In certain embodiments, the formulations of the invention, e.g., the CoQ10 formulations, can be used in combination therapy with at least one additional anticancer agent, e.g., chemotherapeutic agent. In preferred embodiments, CoQ10 is administered in an amount that would be therapeutically effective if delivered alone, i.e., CoQ10 is administered and/or acts as a therapeutic agent, and not predominantly as an agent to ameliorate side effects of other chemotherapy or other cancer treatments. CoQ10 and/or pharmaceutical formulations thereof and the other therapeutic agent can act additively or, more preferably, synergistically. In one embodiment, CoQ10 and/or a formulation thereof is administered concurrently with the administration of an additional anticancer (e.g., chemotherapeutic) agent. In another embodiment, a compound and/or pharmaceutical formulation thereof is administered prior or subsequent to administration of another therapeutic agent wherein both agents are present in the subject at the same time or have therapeutic activity in the subject at the same time. In one embodiment, the CoQ10 and additional anticancer (e.g., chemotherapeutic) agent act synergistically. In one embodiment, the CoQ10 and additional anticancer (e.g., chemotherapeutic) agent act additively.

In one embodiment, the compositions of the invention are for use in therapeutic methods that further comprise administration of one or more additional therapeutic agents, e.g., one or more anticancer agents, e.g., chemotherapeutic agents , e.g., small molecule anticancer agents, biologic anticancer agents including both protein based and nucleic acid based therapeutics. For example, in one embodiment, an additional anticancer agent for use in the invention is a chemotherapeutic agent.

Small molecule chemotherapeutic agents generally belong to various classes including, for example: 1. Topoisomerase II inhibitors (cytotoxic antibiotics), such as the anthracyclines/anthracenediones, *e.g.*, doxorubicin, epirubicin, idarubicin and nemorubicin, the anthraquinones, e.g., mitoxantrone and losoxantrone, and the podophillotoxines, e.g., etoposide and teniposide; 2. Agents that affect microtubule formation (mitotic inhibitors), such as plant alkaloids (e.g., a compound belonging to a family of alkaline, nitrogen-containing molecules derived from plants that are biologically active and cytotoxic), e.g., taxanes, e.g., paclitaxel and docetaxel, and the vinka alkaloids, e.g., vinblastine, vincristine, and vinorelbine, and derivatives of podophyllotoxin; 3. Alkylating agents, such as nitrogen mustards, ethyleneimine compounds, alkyl sulphonates and other compounds with an alkylating action such as nitrosoureas, dacarbazine, cyclophosphamide, ifosfamide and melphalan; 4. Antimetabolites (nucleoside inhibitors), for example, folates, e.g., folic acid, fiuropyrimidines, purine or pyrimidine analogues such as 5-fluorouracil, capecitabine, gemcitabine, methotrexate, and edatrexate; 5. Topoisomerase I inhibitors, such as topotecan, irinotecan, and 9- nitrocamptothecin, camptothecin derivatives, and retinoic acid; and 6. Platinum compounds/complexes, such as cisplatin, oxaliplatin, and carboplatin; Exemplary chemotherapeutic agents for use in the methods of the invention include, but are not limited to, amifostine (ethyol), cisplatin, dacarbazine (DTIC), dactinomycin, mechlorethamine (nitrogen mustard), streptozocin, cyclophosphamide, carmustine (BCNU), lomustine (CCNU), doxorubicin (adriamycin), doxorubicin lipo (doxil), gemcitabine (gemzar), daunorubicin, daunorubicin lipo (daunoxome), procarbazine, mitomycin, cytarabine, etoposide, methotrexate, 5- fluorouracil (5-FU), vinblastine, vincristine, bleomycin, paclitaxel (taxol), docetaxel (taxotere), aldesleukin, asparaginase, busulfan, carboplatin, cladribine, camptothecin, CPT-I1, lO-hydroxy-7-ethyl-camptothecin (SN38), dacarbazine, S-I capecitabine, ftorafur, 5'deoxyflurouridine, UFT, eniluracil, deoxycytidine, 5-azacytosine, 5- azadeoxycytosine, allopurinol, 2-chloro adenosine, trimetrexate, aminopterin, methylene-10-deazaaminopterin (MDAM), oxaplatin, picoplatin, tetraplatin, satraplatin, platinum-DACH, ormaplatin, CI-973, JM-216, and analogs thereof, epirubicin, etoposide phosphate, 9-aminocamptothecin, 10, 11-methylenedioxycamptothecin, karenitecin, 9-nitrocamptothecin, TAS 103, vindesine, L-phenylalanine mustard, ifosphamidemefosphamide, perfosfamide, trophosphamide carmustine, semustine, epothilones A-E, tomudex, 6-mercaptopurine, 6-thioguanine, amsacrine, etoposide phosphate, karenitecin, acyclovir, valacyclovir, ganciclovir, amantadine, rimantadine, lamivudine, zidovudine, bevacizumab, trastuzumab, rituximab, 5-Fluorouracil, Capecitabine, Pentostatin, Trimetrexate, Cladribine, floxuridine, fludarabine, hydroxyurea, ifosfamide, idarubicin, mesna, irinotecan, mitoxantrone, topotecan, leuprolide, megestrol, melphalan, mercaptopurine, plicamycin, mitotane, pegaspargase, pentostatin, pipobroman, plicamycin, streptozocin, tamoxifen, teniposide, testolactone, thioguanine, thiotepa, uracil mustard, vinorelbine, chlorambucil, cisplatin, doxorubicin, paclitaxel (taxol), bleomycin, mTor, epidermal growth factor receptor (EGFR), and fibroblast growth factors (FGF) and combinations thereof which are readily apparent to one of skill in the art based on the appropriate standard of care for a particular tumor or cancer.

In another embodiment, an additional chemotherapeutic agent for use in the combination therapies of the invention is a biologic agent.

Biologic agents (also called biologics) are the products of a biological system, e.g., an organism, cell, or recombinant system. Examples of such biologic agents include nucleic acid molecules (e.g., antisense nucleic acid molecules), interferons, interleukins, colony-stimulating factors, antibodies, e.g., monoclonal antibodies, anti-angiogenesis agents, and cytokines. Exemplary biologic agents are discussed in more detail below and generally belong to various classes including, for example: 1. Hormones, hormonal analogues, and hormonal complexes, e.g., estrogens and estrogen analogs, progesterone, progesterone analogs and progestins, androgens, adrenocorticosteroids, antiestrogens, antiandrogens, antitestosterones, adrenal steroid inhibitors, and anti-leuteinizing hormones; and 2. Enzymes, proteins, peptides, polyclonal and/or monoclonal antibodies, such as interleukins, interferons, colony stimulating factor, etc.

In one embodiment, the biologic is an interfereon. Interferons (IFN) are a type biologic agent that naturally occurs in the body. Interferons are also produced in the laboratory and given to cancer patients in biological therapy. They have been shown to improve the way a cancer patient's immune system acts against cancer cells.

Interferons may work directly on cancer cells to slow their growth, or they may cause cancer cells to change into cells with more normal behavior. Some interferons may also stimulate natural killer cells (NK) cells, T cells, and macrophages which are types of white blood cells in the bloodstream that help to fight cancer cells.

In one embodiment, the biologic is an interleukin. Interleukins (IL) stimulate the growth and activity of many immune cells. They are proteins (cytokines and chemokines) that occur naturally in the body, but can also be made in the laboratory. Some interleukins stimulate the growth and activity of immune cells, such as lymphocytes, which work to destroy cancer cells.

In another embodiment, the biologic is a colony-stimulating factor. Colony-stimulating factors (CSFs) are proteins given to patients to encourage stem cells within the bone marrow to produce more blood cells. The body constantly needs new white blood cells, red blood cells, and platelets, especially when cancer is present. CSFs are given, along with chemotherapy, to help boost the immune system. When cancer patients receive chemotherapy, the bone marrow's ability to produce new blood cells is suppressed, making patients more prone to developing infections. Parts of the immune system cannot function without blood cells, thus colony-stimulating factors encourage the bone marrow stem cells to produce white blood cells, platelets, and red blood cells.

With proper cell production, other cancer treatments can continue enabling patients to safely receive higher doses of chemotherapy.

In another embodiment, the biologic is an antibody. Antibodies, e.g., monoclonal antibodies, are agents, produced in the laboratory, that bind to cancer cells.

Monoclonal antibody agents do not destroy healthy cells. Monoclonal antibodies achieve their therapeutic effect through various mechanisms. They can have direct effects in producing apoptosis or programmed cell death. They can block growth factor receptors, effectively arresting proliferation of tumor cells. In cells that express monoclonal antibodies, they can bring about anti-idiotype antibody formation.

Examples of antibodies which may be used in the combination treatment of the invention include anti-CD20 antibodies, such as, but not limited to, cetuximab, Tositumomab, rituximab, and Ibritumomab. Anti-HER2 antibodies may also be used in combination with CoQ10 for the treatment of cancer. In one embodiment, the anti-HER2 antibody is Trastuzumab (Herceptin). Other examples of antibodies which may be used in combination with CoQ10 for the treatment of cancer include anti-CD52 antibodies (e.g., Alemtuzumab), anti-CD-22 antibodies (e.g., Epratuzumab), and anti-CD33 antibodies (e.g., Gemtuzumab ozogamicin). Anti-VEGF antibodies may also be used in combination with CoQ10 for the treatment of cancer. In one embodiment, the anti-VEGF antibody is bevacizumab. In other embodiments, the biologic agent is an antibody which is an anti-EGFR antibody e.g., cetuximab. Another example is the anti-glycoprotein 17-1A antibody edrecolomab. Numerous other anti-tumor antibodies are known in the art and would be understood by the skilled artisan to be encompassed by the present invention.

In another embodiment, the biologic is a cytokine. Cytokine therapy uses proteins (cytokines) to help a subject's immune system recognize and destroy those cells that are cancerous. Cytokines are produced naturally in the body by the immune system, but can also be produced in the laboratory. This therapy is used with advanced melanoma and with adjuvant therapy (therapy given after or in addition to the primary cancer treatment). Cytokine therapy reaches all parts of the body to kill cancer cells and prevent tumors from growing.

In another embodiment, the biologic is a fusion protein. For example, recombinant human Apo2L/TRAIL (GENETECH) may be used in a combination therapy. Apo2/TRAIL is the first dual pro-apoptotic receptor agonist designed to activate both pro-apoptotic receptors DR4 and DR5, which are involved in the regulation of apoptosis (programmed cell death).

In one embodiment, the biologic is a therapeutic nucleic acid molecule. Nucleic acid therapeutics are well known in the art. Nucleic acid therapeutics include both single stranded and double stranded (i.e., nucleic acid therapeutics having a complementary region of at least 15 nucleotides in length) nucleic acids that are complementary to a target sequence in a cell. Therapeutic nucleic acids can be directed against essentially any target nucleic acid sequence in a cell. In certain embodiments, the nucleic acid therapeutic is targeted against a nucleic acid sequence encoding a stimulator of angiogenesis, e.g., VEGF, FGF, or of tumor growth, e.g., EGFR.

Antisense nucleic acid therapeutic agents are single stranded nucleic acid therapeutics, typically about 16 to 30 nucleotides in length, and are complementary to a target nucleic acid sequence in the target cell, either in culture or in an organism.

In another aspect, the agent is a single-stranded antisense RNA molecule. An antisense RNA molecule is complementary to a sequence within the target mRNA. Antisense RNA can inhibit translation in a stoichiometric manner by base pairing to the mRNA and physically obstructing the translation machinery, see Dias, N. et al., (2002) Mol Cancer Ther 1:347-355. The antisense RNA molecule may have about 15-30 nucleotides that are complementary to the target mRNA. Patents directed to antisense nucleic acids, chemical modifications, and therapeutic uses are provided, for example, in U.S. Patent No. 5,898,031 related to chemically modified RNA-containing therapeutic compounds, and U.S. Patent No. 6,107,094 related methods of using these compounds as therapeutic agent. U.S. Patent No. 7,432,250 related to methods of treating patients by administering single-stranded chemically modified RNA-like compounds; and U.S. Patent No. 7,432,249 related to pharmaceutical compositions containing single-stranded chemically modified RNA-like compounds. U.S. Patent No. 7,629,321 is related to methods of cleaving target mRNA using a single-stranded oligonucleotide having a plurality RNA nucleosides and at least one chemical modification. The entire contents of each of the patents listed in this paragraph are incorporated herein by reference.

Nucleic acid therapeutic agents for use in the methods of the invention also include double stranded nucleic acid therapeutics. An "RNAi agent," "double stranded RNAi agent," double-stranded RNA (dsRNA) molecule, also referred to as "dsRNA agent," "dsRNA", "siRNA", "iRNA agent," as used interchangeably herein, refers to a complex of ribonucleic acid molecules, having a duplex structure comprising two antiparallel and substantially complementary, as defined below, nucleic acid strands. As used herein, an RNAi agent can also include dsiRNA (see, e.g., US Patent publication 20070104688). In general, the majority of nucleotides of each strand are ribonucleotides, but as described herein, each or both strands can also include one or more non-ribonucleotides, e.g., a deoxyribonucleotide and/or a modified nucleotide. In addition, as used in this specification, an "RNAi agent" may include ribonucleotides with chemical modifications; an RNAi agent may include substantial modifications at multiple nucleotides. Such modifications may include all types of modifications disclosed herein or known in the art. Any such modifications, as used in a siRNA type molecule, are encompassed by "RNAi agent" for the purposes of this specification and claims. The RNAi agents that are used in the methods of the invention include agents with chemical modifications as disclosed, for example, in U.S. Provisional Application No. 61/561,710, filed on November 18, 2011, International Application No. PCT/US2011/051597, filed on September 15, 2010, and PCT Publication WO 2009/073809.

Additional exemplary biologic agents for use in the methods of the invention include, but are not limited to, gefitinib (Iressa), anastrazole, diethylstilbesterol, estradiol, premarin, raloxifene, progesterone, norethynodrel, esthisterone, dimesthisterone, megestrol acetate, medroxyprogesterone acetate, hydroxyprogesterone caproate, norethisterone, methyltestosterone, testosterone, dexamthasone, prednisone, Cortisol, solumedrol, tamoxifen, fulvestrant, toremifene, aminoglutethimide, testolactone, droloxifene, anastrozole, bicalutamide, flutamide, nilutamide, goserelin, flutamide, leuprolide, triptorelin, aminoglutethimide, mitotane, goserelin, cetuximab, erlotinib, imatinib, Tositumomab, Alemtuzumab, Trastuzumab, Gemtuzumab, Rituximab, Ibritumomab tiuxetan, Bevacizumab, Denileukin diftitox, Daclizumab, interferon alpha, interferon beta, anti-4-1BB, anti-4-lBBL, anti-CD40, anti-CD 154, anti-OX40, anti-OX40L, anti-CD28, anti-CD80, anti-CD86, anti-CD70, anti-CD27, anti-HVEM, anti-LIGHT, anti-GITR, anti-GITRL, anti-CTLA-4, soluble OX40L, soluble 4-IBBL, soluble CD154, soluble GITRL, soluble LIGHT, soluble CD70, soluble CD80, soluble CD86, soluble CTLA4-Ig, GVAX®, and combinations thereof which are readily apparent to one of skill in the art based on the appropriate standard of care for a particular tumor or cancer. The soluble forms of agents may be made as, for example fusion proteins, by operatively linking the agent with, for example, Ig-Fc region.

It should be noted that more than one additional anticancer agents (e.g., chemotherapeutic agents), *e.g.,* 2, 3, 4, 5, or more, may be administered in combination with the CoQ10 formulations provided herein. For example, in one embodiment two additional chemotherapeutic agents may be administered in combination with CoQ10. For example, in one embodiment, a chemotherapeutic small molecule agent, a chemotherapeutic biologic agent, and CoQ10 may be administered. Appropriate doses and routes of administration of the chemotherapeutic agents provided herein are known in the art.

Reference will now be made in detail to preferred embodiments of the invention. While the invention will be described in conjunction with the preferred embodiments, it will be understood that it is not intended to limit the invention to those preferred embodiments. To the contrary, it is intended to cover alternatives, modifications, and equivalents as may be included within the scope of the invention as defined by the appended claims.

### EXAMPLES

### EXAMPLE 1 - Treatment of Cancer in Humans with Coenzyme Q10 After Failure with Chemotherapeutic Agents

A Phase I trial of Coenzyme Q10 (IV formulation C31510) was performed to determine the maximum tolerated dose (MTD), the safety/pharmacodynamics (PK) correlates, and commence exploratory pharmacodynamics (PD). The study was also performed to examine preliminary efficacy (using RECIST 1.1 criteria), overall clinical benefit, and progression free survival. An interim analysis was performed on the study participants.

### Study population

### Age (mean) = 54

### Male/Female 14/17

3 Stage IV Pancreatic Cancers
3 Stage IV Uterine Sarcomas
3 Stage IV Myxoid Liposarcomas
3 Stage IV Leiomyosarcomas
2 Stage IV Chondrosarcomas
2 Stage IV Osteosarcomas
2 Angiosarcoma
1 Stage IV Adenocarcinoma of Colon
1 Stage IV Squamous Cell of Cervix
1 Stage IV Squamous Cell of Tonsil
1 Stage IV Papillary Thyroid
1 Stage IV Adenoid Cystic
1 Stage IV Synovial Cell Sarcoma
1 Stage IV Malignant fibrous histiocytoma
1 Stage IV Desmoplastic Sarcoma
1 Stage IV Hepatocellular Carcinoma
1 Stage IV Spindle Cell Sarcoma
1 Stage III Cholangiocarcinoma
1 Appendiceal Carcinoma
1 Pleiomorphic Sarcoma

All of the patients in this initial group had advanced disease which had been refractory to one or more chemotherapeutic regimens at time of enrollment.

Recruitment and treatment were ongoing at the time of this interim analysis, with 42 subjects enrolled in the study. The results in this example are for the 31 subjects listed above.

### Study Design

A standard 3 x 3 phase I dose-escalation design was used with patients (n=31) with advanced solid malignancies. CoQ10 was delivered as a sterile nanosuspension in the preferred intravenous formulation provided herein using the following dose escalation design:

| **Cohort** | **Dosing of CoQ10** | **Volume of Normal Saline** | **Drug Product for 70 kg Subject** |
|---|---|---|---|
| 1 | 5.62 mg/kg | 100 ml | 9.84 ml |
| 2 | 11.25 mg/kg | 250 ml | 19.7 ml |
| 3 | 22.5 mg/kg | 500 ml | 39.4 ml |
| 4 | 33.0 mg/kg | 500 ml | 58.7 ml |
| 5 | 44.0 mg/kg | 500 ml | 77.0 ml |
| 6 | 58.7 mg/kg | 500 ml | 102.7 ml |
| 7 | 78.2 mg/kg | 500 ml | 136.6 ml |
| 8 | 104.3 mg/kg | 500 ml | 182.5 ml |
| 9 | 139 mg/kg | NA | 243.3 ml |

In the study, coenzyme Q10 was administered as a four-hour IV infusion three days a week for 28 days (one cycle) until clinical progression. Pharmacokinetic (PK) information was calculated with serial serum collection data. Tumor response was determined using RECIST 1.1. Clinical benefit (decreased pain, increased quality of life) was assessed by the treating physician.

### Toxicities

No significant (i.e., Grade III or Grade IV) toxicities were observed at the time of the analysis. No dose limiting toxicities have been observed and the maximum tolerated dose (MTD) has not been identified. At the first interim analysis, the highest dose administered was 104.3 mg/kg per dose.

As of the first interim analysis, minor toxicities that were easily addressed were observed. Specifically, 23 (74.1%) patients developed a Grade I asymptomatic elevation of INR and 14 (45.1 %) patients had a Grade II INR elevation of greater than 2.0; four subjects (12.9%) had an INR greater than 3 and two (6%) subjects had an INR greater than 5 which did not result in withdrawal from the trial. In all subject, INR was quickly normalized with one intramuscular injection of Vitamin K. Nine (29.0%) of patients reported having a headache during first week of treatment relieved by acetaminophen.

### Population Outcomes- Interim Analysis

The 42 patients enrolled, all with advanced solid malignancies, were assigned to 8 dose cohorts (5.62 mg/kg, 11.25 mg/kg, 22.5 mg/kg, 33 mg/kg, 44 mg/kg, 58.7 mg/kg, 78.2 mg/kg, 104.3 mg/kg). As of the first analysis, no subjects had been dosed at the maximum planned dose of 139 mg/kg. The trial was still ongoing and recruiting subjects at the first interim analysis, and a MTD had not been reached. At the time of this analysis, 31 (73.8%) of subjects successfully completed at least one cycle and are considered to be evaluable.

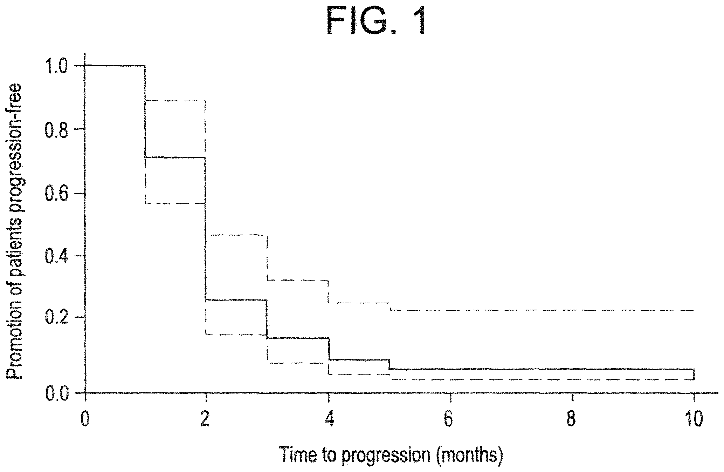

Subjects received a median of 2 cycles (1-10) and remained on treatment for an average of 90 days. Median Progression Free survival was 2 months (Figure 1). Nineteen (61.2%) patients had a clinical benefit and remained on treatment for an average of 16 weeks. These results demonstrate the efficacy of CoQ10 in the treatment of cancer in subjects who have failed more than one chemotherapeutic regimen.

### Individual Patient Outcomes

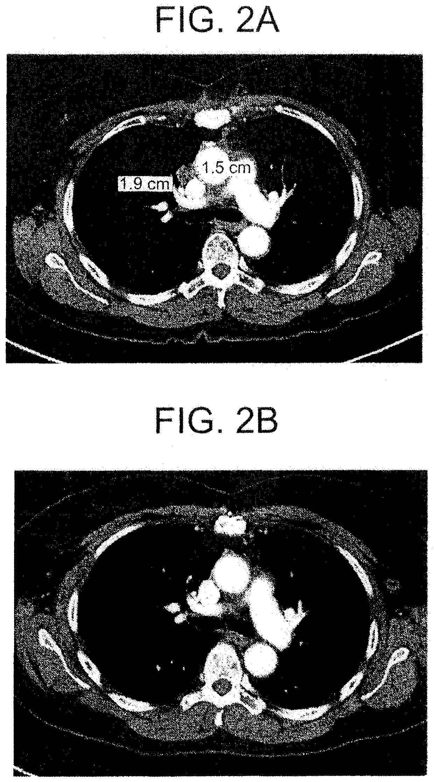

A 62-year-old woman with myxoid liposarcoma with metastatic disease to the mediastinum, heart, and lungs was treated with the at the 58.6 mg/kg/dose. Prior treatment regimens had included adriamycin, ifosfamide, etoposide, vincristine, gemzar, and taxotere. Four treatment cycles (four-hour IV infusion three days a week for 28 days (one cycle)) with coenzyme Q10 resulted in resolution of her anterior mediastinal lesion. Before and after images are provided in Figures 2A and B, respectively.

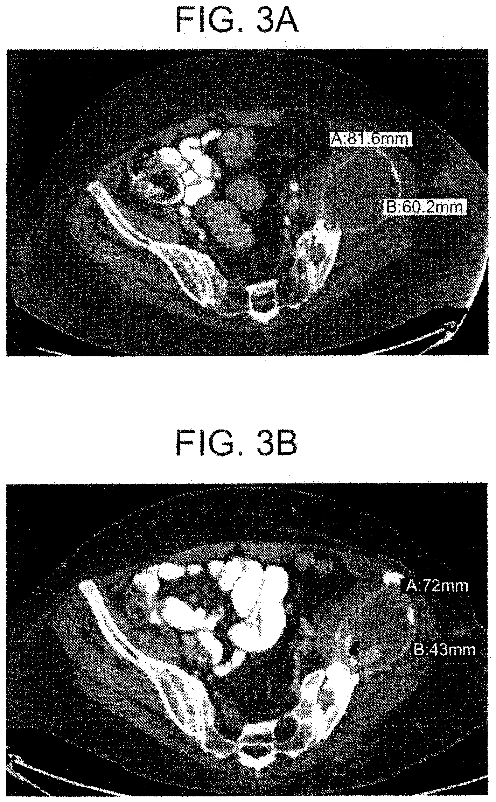

A 62-year-old woman with pleomorphic fibrosarcoma of the left ilium with diffuse bone metastasis who progressed through treatments with Th-302 + Adriamycin after 7 cycles was enrolled in the trial. She had a minor response to an escalating dose of CoQ10 after 6 cycles of escalating doses (from 22.5 mg/kg/dose to 58.7 mg/kg/dose) with significant improvement in pain. Tumor shrinkage was also observed as shown in Figures 3A and B. The response lasted over 10 months.

### Summary

Interim data from this Phase I study indicates that coenzyme Q10 is well tolerated with no dose limiting toxicity to date. An asymptomatic elevation in the INR and headaches during the first week of treatment were commonly observed. There were no grade 3/4 toxicities. At the time of this analysis, the trial was still open and no MTD had been reached. A partial response and minor response were observed and a majority of patients enrolled in this trial had an increased progression free survival and/or clinical benefit. Taken together, there is a compelling rationale for clinical development of CoQ10 for treatment of solid tumors. These results demonstrate the efficacy of CoQ10 in the treatment of cancer in subjects who have failed more than one chemotherapeutic regimen.

### EXAMPLE 2 - Pharmacokinetic Analysis

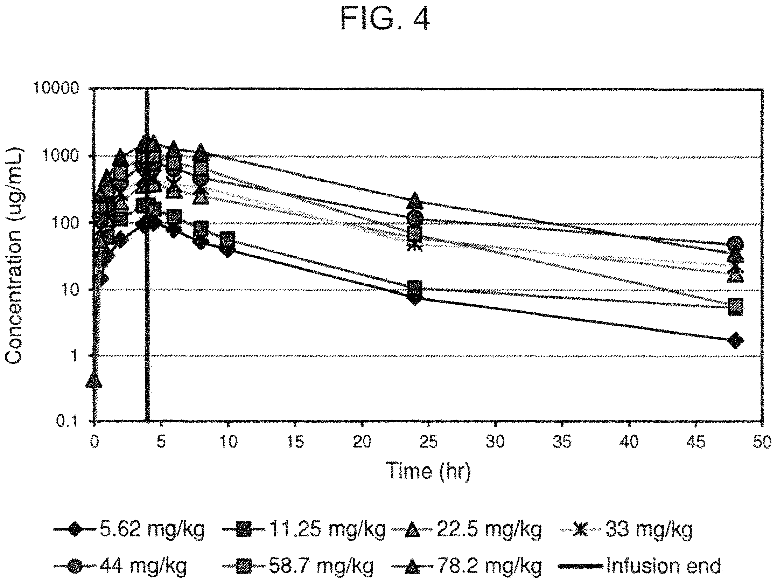

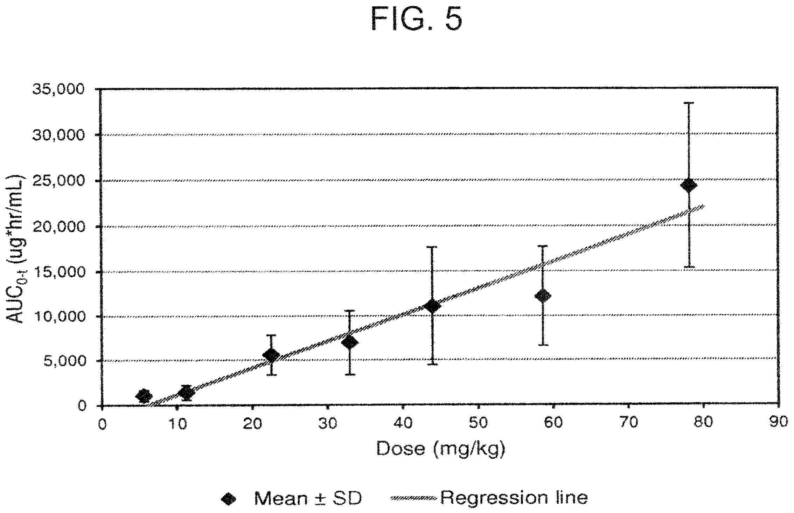

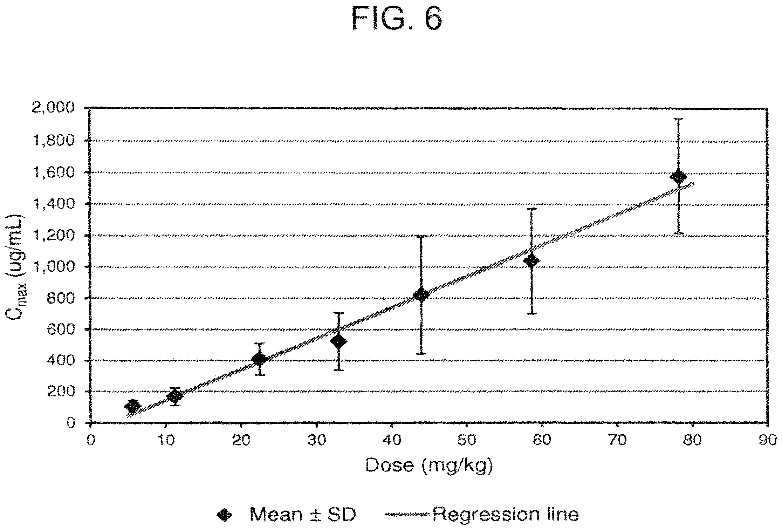

Pharmacokinetic and pharmacodynamic analyses were performed during the study as well. The pharmacokinetics is linear (Figure 4). Tₘₐₓ and Cₘₐₓ were associated with the end of the infusion. The t_{1/2} ranged from 2.18 to 13.3 hr, with little or no dependence on dose (see Figure 5). There were no sex differences in the parameters normalized by dose and body surface area (Figure 6).

### EXAMPLE 3 - Ongoing Analysis of Outcomes of Treatment of Cancer in Humans with Coenzyme Q10 after Failure with Chemotherapeutic Agents

Recruitment, treatment, and analysis continued after the first interim analysis provided in Example 1. A total of 63 potential subjects were identified, 50 of whom entered the trial. Characteristics of the total population of subjects that entered the trial were as follows:
All subjects had solid tumors.
Male/female: 25/25
Median Age (range): 56 (19-94)
White: 42
Asian: 6
Black: 1
Native Hawaiian: 1

The disease characteristics of total population of subjects who entered the trial are presented in the table below. The majority had sarcomas involving an extremity; other tumor types included hepatic/biliary, pancreas, and uterine carcinomas. Most subjects had Stage IV advanced (44%) and/or metastatic (84%) disease. Nearly all subjects had prior surgery (98%) and/or radiation.

| **Parameter** | **Data** | **n** | **%** |
|---|---|---|---|
| Primary Tumor Site | Extremity | 17 | 34% |
| | Hepatic/Biliary | 5 | 10% |
| | Pancreas | 4 | 8% |
| | Uterus | 4 | 8% |
| Histology | Sarcoma | 31 | 62% |
| | Adenocarcinoma | 9 | 18% |
| | Carcinoma | 8 | 16% |
| Stage at Study Entry | I | 3 | 6% |
| | II | 15 | 30% |
| | III | 7 | 14% |
| | IV | 22 | 44% |
| | Unknown | 3 | 6% |
| Metastatic Disease | No | 8 | 16% |
| | Yes | 42 | 84% |
| Number of Prior Therapies | 0 | 1 | 2% |
| | 1-3 | 24 | 48% |
| | 4-6 | 20 | 40% |
| | 8-10 | 5 | 10% |
| Best Response to Prior Treatment* | Complete response | 12 | 6% |
| | Partial Response | 3 | 1.5% |
| | Stable Disease | 119 | 61% |
| | Progressive Disease | 44 | 23% |
| | Unknown | 13 | 7% |
| | Not Available | 3 | 1.5% |
| Reason Off Prior Treatment* | Progressive Disease | 80 | 41% |
| | Completed Regimen | 78 | 40% |
| | Toxicity | 32 | 16% |
| | Other | 4 | 2% |
| Prior Radiation | No | 16 | 32% |
| | Yes | 34 | 68% |
| | - Pallative | 48 | 69% |
| | - Adjuvant | 14 | 20% |
| | - Curative | 7 | 10% |
| Prior Surgery^{#} | No | 1 | 2% |
| | Yes | 49 | 98% |
| | - Diagnostic | 77 | 38% |
| | - Palliative | 68 | 34% |
| | - Curative | 56 | 28% |

| | | | |
|---|---|---|---|
| * As most subjects were treated with multiple prior therapies, the number of responses is greater than the number of subjects enrolled. ^{#} As many subjects had undergone multiple surgical procedures, the number of surgeries is greater than the number of subjects enrolled. | | | |

An exploratory assessment of antitumor activity was undertaken during the study using modified Response Criteria in Solid Tumors (RECIST) v1.1. Tumors were measured at baseline or screening, at the end of Cycle 1 and Cycle 2 treatments, and once every 2 treatment cycles (2 months) thereafter in the absence of clinically rapid progression of disease.

In the study, 50 subjects were exposed to 9 dosages of CoQ10 ranging from 5.62 mg/kg to 139 mg/kg. For the population as a whole, the median duration of treatment was 7.29 weeks (range: 0.29 - 53.6 weeks). The majority of subjects (52%) completed Cycle 1 (treated 3 times per week for 4 weeks).

Thirteen (13) subjects escalated from their initial assigned dose level to a higher dosage of the study drug after demonstrating acceptable tolerability of CoQ10 in the nanosuspension formulation. Only 1 subject in Cohort 9 (139 mg/kg) had a dose reduction due to grade 3 infection of an open wound present at study entry on the tibia. The subject missed 9 doses of study drug while hospitalized for antibiotics. The CoQ10 formulation was resumed at a reduced dose of 104.3 mg/kg for 8 doses before the subject discontinued treatment due to the Sponsor-initiated study hold. Treatment exposures to the CoQ10 formulation during the study are summarized in the table.

| | **Completed C1** | | | **Dose Reduced** | |
|---|---|---|---|---|---|
| | Yes | % | n=50 | Yes | No |
| Cohort 1 - 5.62 mg/kg (n=3) | 2 | 67 | 4 | 0 | 3 |
| Cohort 2 - 11.25 mg/kg (n=6) | 5 | 83 | 10 | 0 | 6 |
| Cohort 3 - 22.5 mg/kg (n=7) | 6 | 86 | 12 | 0 | 7 |
| Cohort 4 - 33.0 mg/kg (n=6) | 3 | 50 | 6 | 0 | 6 |
| Cohort 5 - 44.0 mg/kg (n=5) | 3 | 60 | 6 | 0 | 5 |
| Cohort 6 - 58.7 mg/kg (n=3) | 2 | 67 | 4 | 0 | 3 |
| Cohort 7 - 78.2 mg/kg (n=7) | 1 | 14 | 2 | 0 | 6 |
| Cohort 8 - 104.3 mg/kg (n=8) | 4 | 50 | 8 | 0 | 10 |
| Cohort 9 - 139 mg/kg (n=5) | 0 | 0 | 0 | 1 | 3 |

A plurality of subjects (23, 46%) had a best response to intravenous administration of the nanosuspension of CoQ10 of stable disease. Stable disease was achieved in a variety of tumor types, including: pancreas, colon, rectum, bile duct, oral, and liver carcinomas as well as uterus, extremity, and retroperitoneal sarcomas. One subject (2%) with sarcoma of the extremity had a partial response lasting at least 6 months and did not have documented progression prior to voluntarily withdrawing from the study. Fifteen subjects (30%) had a best response of disease progression. The best response of subjects is summarized in the table below:

| **Best Response** | **n** | **%** |
|---|---|---|
| Progressive Disease | 15 | 30% |
| Stable Disease | 23 | 46% |
| Partial Response | 1 | 2% |
| Complete Response | 0 | 0% |
| Unknown | 1 | 2% |
| Unevaluable | 10 | 20% |

Subjects discontinued the study for a variety of reasons which are summarized in the table below.

| **Primary Reason for Discontinuation** | **n** | **%** |
|---|---|---|
| Intolerable Toxicity | 4 | 8% |
| Personal Preference of the Patient for Any Reason | 8 | 16% |
| Intercurrent Illness | 3 | 6% |
| Progressive Disease | 26 | 52% |
| Unknown | 5 | 10% |
| Sponsor Safety Hold | 3 | 6% |

These result demonstrate that the majority of evaluable subjects (60%, 24 of 40 evaluable subjects, 23 with at least stable disease, one with at least a partial response) who completed at least one treatment cycle with CoQ10 achieved a clinically relevant response by RECIST 1.1 criteria during the study. This outcome is especially notable as all of the subjects had failed at least one prior chemotherapeutic regimen, nearly all of the subjects had been treated with at least one surgical intervention, and many had undergone prior radiation treatments. Moreover, due to the dose escalation format of the trial, some of the subjects in the trial received lower doses of CoQ10 than were found to be tolerable.

### EXAMPLE 4 -Analysis of Outcomes of Treatment of Cancer in Humans with Coenzyme Q10 after Failure with Chemotherapeutic Agents

Subjects were categorized based on the best response in the clinical trial. Of the 50 subjects enrolled in the study, 39 subjects had evaluable outcomes, 10 subjects had unevaluable outcomes, and one subject had an unknown outcome. Of the 39 evaluable subjects, 2 achieved a best outcome of partial response, 22 achieved a best outcome of stable disease, and 15 achieved a best outcome of progressive disease. It is notable that both subjects who achieved a partial response had stage IV disease. The number of treatments prior to the study (range, median and average) and the number of doses received in the study (range, median and average) were calculated for the evaluable subjects and are provided in the table below.

| **Best response** | **# Prior treatments with Other Regimens** | | | **# Doses Received in Study** | | |
|---|---|---|---|---|---|---|
| | Range | Median | Average | Range | Median | Average |
| Clinical progression (n=15) | 2-10 | 3 | 4.1 | 4-34 | 12 | 15.1 |
| Stable Disease (n=22) | 1-8 | 4-5 | 3.8 | 12-81 | 26-28 | 33.8 |
| Partial Response (n=2) | 1-2 | NA | 1.5 | 66-123 | NA | 94.5 |

## Claims

1. A composition for use in treating a cancer in a subject, wherein the subject has failed treatment for the cancer with at least one chemotherapeutic regimen, the composition comprising coenzyme Q10 (CoQ10), wherein the cancer is selected from the group consisting of colon cancer, rectal cancer, pancreatic cancer, liver cancer, oral cancer, uterine cancer and myxoid liposarcoma.

2. The composition for use of claim 1, wherein the subject has failed treatment for the cancer with at least two previous chemotherapeutic regimens.

3. The composition for use of claim 1, wherein the cancer is a refractory cancer.

4. The composition for use of claim 1, wherein failure with at least one chemotherapeutic regimen comprises tumor growth during or after treatment with the chemotherapeutic regimen.

5. The composition for use of claim 1, wherein failure with at least one chemotherapeutic regimen comprises a dose limiting toxicity due to the chemotherapeutic regimen.

6. The composition for use of any one of claims 1 to 5, wherein the subject demonstrates one or more clinical benefits as a result of administration of the CoQ10, and wherein the one or more clinical benefits is selected from the group consisting of stable disease per RECIST 1.1 criteria, partial response per RECIST 1.1 criteria, and complete response per RECIST 1.1 criteria.

7. The composition for use of claim 1, wherein the subject does not exhibit a dose limiting toxicity in response as a result of administration of the CoQ10.

8. The composition for use of claim 1, wherein the cancer comprises a Stage III tumor.

9. The composition for use of claim 1, wherein the cancer comprises a Stage IV tumor.

10. The composition for use of claim 1, wherein the cancer is metastatic.

11. The composition for use of claim 1, wherein the cancer is a cancer selected from the group consisting of colon cancer, rectal cancer, pancreatic cancer, and liver cancer.

12. The composition for use of claim 1, wherein the cancer is a cancer selected from the group consisting of: pancreatic cancer, uterine cancer, myxoid liposarcoma, colon cancer, and liver cancer.

13. The composition for use of claim 1, wherein the subject has failed treatment with a chemotherapeutic regimen comprising at least one chemotherapeutic agent selected from the group consisting of adriamycin, ifosfamide, etoposide, vincristine, gemzar, taxotere, and Th-302.

14. The composition for use of claim 1, wherein the subject has failed treatment with a chemotherapeutic regimen comprising at least one chemotherapeutic agent selected from the group consisting of a topoisomerase I inhibitor, a topoisomerase II inhibitor, a mitotic inhibitor, an alkylating agent, a platinum compound, and an antimetabolite.

15. The composition for use of any one of claims 1 to 14, wherein the CoQ10 is administered at a dose selected from the group consisting of at least 5.6 mg/kg/dose, at least 11.2 mg/kg/dose, at least 22.5 mg/kg/dose, at least 33 mg/kg/dose, at least 44 mg/kg/dose, at least 50 mg/kg/dose, at least 58.7 mg/kg/dose, at least 75 mg/kg/dose, at least 78.2 mg/kg/dose, at least 100 mg/kg/dose, at least 104.3 mg/kg/dose, at least 125 mg/kg/dose, at least 139 mg/kg/dose, at least 150 mg/kg/dose, and at least 200 mg/kg/dose.

16. The composition for use of claim 1, wherein at least 12, 15, 26, or 33 doses of the composition are administered to the subject.

17. The composition for use of claim 1, wherein the subject has failed 8 or fewer chemotherapeutic regimens or 5 or fewer chemotherapeutic regimens.

18. The composition for use of claim 1, wherein the composition is administered by injection or infusion.

19. The composition for use of claim 1, wherein the subject is human.

20. The composition for use of claim 1, wherein the CoQ10 is formulated as a nanodispersion.

21. The composition for use of claim 1, wherein the CoQ10 is provided for intravenous administration in a CoQ10 formulation comprising:
an aqueous solution;
a CoQ10 dispersed into a nano-dispersion of particles; and
at least one of a dispersion stabilizing agent and an opsonization reducer;
wherein the nano-dispersion of the CoQ10 is dispersed into nano-particles having a mean particle size of less than 200 nm.

22. The composition for use of claim 1, wherein the composition is administered to the subject with an additional agent.

23. The composition for use of claim 22, wherein the additional agent is a chemotherapeutic agent, small molecule anticancer agent, or biologic anticancer agent including both protein based and nucleic acid based therapeutics.

24. The composition for use of claim 1, wherein the cancer is pancreatic cancer.

25. The composition for use of claim 23, wherein the additional agent is gemcitabine.

## Patentansprüche

1. Zusammensetzung zur Verwendung beim Behandeln eines Krebses bei einem Probanden, wobei bei dem Probanden eine Behandlung für den Krebs mit mindestens einem chemotherapeutischen Schema fehlgeschlagen ist, wobei die Zusammensetzung Coenzym Q10 (CoQlO) umfasst, wobei der Krebs ausgewählt ist aus der Gruppe bestehend aus Dickdarmkrebs, Mastdarmkrebs, Bauchspeicheldrüsenkrebs, Leberkrebs, Mundkrebs, Gebärmutterkrebs und myxoidem Liposarkom.

2. Zusammensetzung zur Verwendung nach Anspruch 1, wobei bei dem Probanden eine Behandlung für den Krebs mit mindestens zwei vorherigen chemotherapeutischen Schemen fehlgeschlagen ist.

3. Zusammensetzung zur Verwendung nach Anspruch 1, wobei der Krebs ein refraktärer Krebs ist.

4. Zusammensetzung zur Verwendung nach Anspruch 1, wobei das Fehlschlagen mit mindestens einem chemotherapeutischen Schema ein Tumorwachstum während oder nach der Behandlung mit dem chemotherapeutischen Schema umfasst.

5. Zusammensetzung zur Verwendung nach Anspruch 1, wobei das Fehlschlagen mit mindestens einem chemotherapeutischen Schema eine dosislimitierende Toxizität aufgrund des chemotherapeutischen Schemas umfasst.

6. Zusammensetzung zur Verwendung nach einem der Ansprüche 1 bis 5, wobei der Proband eine oder mehrere klinische Nutzwirkungen als ein Ergebnis der Verabreichung des CoQlO demonstriert, und wobei die eine oder mehrere klinische Nutzwirkungen ausgewählt ist aus der Gruppe bestehend aus stabiler Erkrankung gemäß RECIST 1.1-Kriterien, teilweises Ansprechen gemäß RECIST 1.1-Kriterien und vollständiges Ansprechen gemäß RECIST 1.1-Kriterien.

7. Zusammensetzung zur Verwendung nach Anspruch 1, wobei der Proband beim Ansprechen keine dosislimitierende Toxizität als ein Ergebnis der Verabreichung des CoQlO vorweist.

8. Zusammensetzung zur Verwendung nach Anspruch 1, wobei der Krebs einen Stadium III-Tumor umfasst.

9. Zusammensetzung zur Verwendung nach Anspruch 1, wobei der Krebs einen Stadium IV-Tumor umfasst.

10. Zusammensetzung nach Anspruch 1, wobei der Krebs metastasenbildend ist.

11. Zusammensetzung zur Verwendung nach Anspruch 1, wobei der Krebs ein Krebs ist, ausgewählt aus der Gruppe bestehend aus Dickdarmkrebs, Mastdarmkrebs, Bauchspeicheldrüsenkrebs und Leberkrebs.

12. Zusammensetzung zur Verwendung nach Anspruch 1, wobei der Krebs ein Krebs ist, ausgewählt aus der Gruppe bestehend aus: Bauchspeicheldrüsenkrebs, Gebärmutterkrebs, myoxidem Liposarkom, Dickdarmkrebs und Leberkrebs.

13. Zusammensetzung zur Verwendung nach Anspruch 1, wobei bei dem Probanden eine Behandlung mit einem chemotherapeutischen Schema, umfassend mindestens ein chemotherapeutisches Mittel, ausgewählt aus der Gruppe bestehend aus Adriamycin, Ifosfamid, Etoposid, Vincristin, Gemzar, Taxoter und Th-302, fehlgeschlagen ist.

14. Zusammensetzung zur Verwendung nach Anspruch 1, wobei bei dem Probanden eine Behandlung mit einem chemotherapeutischen Schema, umfassend mindestens ein chemotherapeutisches Mittel, ausgewählt aus der Gruppe bestehend aus einem Topoisomerase I-Inhibitor, einem Topoisomerase II-Inhibitor, einem mitotischen Inhibitor, einem Alkylierungsmittel, einer Platinverbindung und einem Antimetaboliten, fehlgeschlagen ist.

15. Zusammensetzung zur Verwendung nach einem der Ansprüche 1 bis 14, wobei das CoQlO bei einer Dosis verabreicht wird, ausgewählt aus der Gruppe bestehend aus mindestens 5,6 mg/kg/Dosis, mindestens 11,2 mg/kg/Dosis, mindestens 22,5 mg/kg/Dosis, mindestens 33 mg/kg/Dosis, mindestens 44 mg/kg/Dosis, mindestens 50 mg/kg/Dosis, mindestens 58,7 mg/kg/Dosis, mindestens 75 mg/kg/Dosis, mindestens 78,2 mg/kg/Dosis, mindestens 100 mg/kg/Dosis, mindestens 104,3 mg/kg/Dosis, mindestens 125 mg/kg/Dosis, mindestens 139 mg/kg/Dosis, mindestens 150 mg/kg/Dosis und mindestens 200 mg/kg/Dosis.

16. Zusammensetzung zur Verwendung nach Anspruch 1, wobei dem Probanden mindestens 12, 15, 26 oder 33 Dosen der Zusammensetzung verabreicht werden.

17. Zusammensetzung zur Verwendung nach Anspruch 1, wobei bei dem Probanden 8 oder weniger chemotherapeutische Schemen oder 5 oder weniger chemotherapeutische Schemen fehlgeschlagen sind.

18. Zusammensetzung zur Verwendung nach Anspruch 1, wobei die Zusammensetzung durch Injektion oder Infusion verabreicht wird.

19. Zusammensetzung zur Verwendung nach Anspruch 1, wobei der Proband ein Mensch ist.

20. Zusammensetzung zur Verwendung nach Anspruch 1, wobei das CoQlO als eine Nanodispersion formuliert ist.

21. Zusammensetzung zur Verwendung nach Anspruch 1, wobei das CoQlO zur intravenösen Verabreichung in einer CoQ10-Formulierung bereitgestellt wird, umfassend:
eine wässrige Lösung;
ein zu einer Nanodispersion von Partikeln dispergiertes CoQlO; und
mindestens ein dispersionsstabilisierendes Mittel und einen Opsonisierungsreduzierer;
wobei die Nanodispersion des CoQlO zu Nanopartikeln mit einer mittleren Partikelgröße von weniger als 200 nm dispergiert wird.

22. Zusammensetzung zur Verwendung nach Anspruch 1, wobei die Zusammensetzung dem Probanden mit einem zusätzlichen Mittel verabreicht wird.

23. Zusammensetzung zur Verwendung nach Anspruch 22, wobei das zusätzliche Mittel ein chemotherapeutisches Mittel, niedermolekulares Antikrebsmittel oder biologisches Antikrebsmittel ist, einschließlich sowohl proteinbasierter als auch nukleinsäurebasierter Therapeutika.

24. Zusammensetzung zur Verwendung nach Anspruch 1, wobei der Krebs Bauchspeicheldrüsenkrebs ist.

25. Zusammensetzung zur Verwendung nach Anspruch 23, wobei das zusätzliche Mittel Gemcitabin ist.

## Revendications

1. Composition à utiliser dans le traitement d'un cancer chez un sujet, dans laquelle le sujet n'a pas réagi à un traitement du cancer avec au moins un régime chimiothérapeutique, la composition comprenant la coenzyme Q10 (CoQlO), dans laquelle le cancer est choisi dans le groupe constitué par le cancer du côlon, le cancer du rectum, le cancer du pancréas, le cancer du foie, le cancer de la bouche, le cancer de l'utérus et le liposarcome myxoïde.

2. Composition à utiliser selon la revendication 1, dans laquelle le sujet n'a pas réagi à un traitement du cancer avec au moins deux régimes chimiothérapeutiques antérieurs.

3. Composition à utiliser selon la revendication 1, dans laquelle le cancer est un cancer réfractaire.

4. Composition à utiliser selon la revendication 1, dans laquelle un échec avec au moins un régime chimiothérapeutique comprend une croissance tumorale pendant ou après un traitement avec le régime chimiothérapeutique.

5. Composition à utiliser selon la revendication 1, dans laquelle un échec avec au moins un régime chimiothérapeutique comprend une toxicité dose limitante due au régime chimiothérapeutique.

6. Composition à utiliser selon l'une quelconque des revendications 1 à 5, dans laquelle le sujet démontre un ou plusieurs avantages cliniques à la suite de l'administration de la CoQlO, et dans laquelle les un ou plusieurs avantages cliniques sont choisis dans le groupe constitué par une maladie stable selon les critères RECIST 1.1, une réponse partielle selon les critères RECIST 1.1 et une réponse complète selon les critères RECIST 1.1.

7. Composition à utiliser selon la revendication 1, dans laquelle le sujet ne présente pas de toxicité dose limitante en réponse à l'administration du CoQlO.

8. Composition à utiliser selon la revendication 1, dans laquelle le cancer comprend une tumeur de stade III.

9. Composition à utiliser selon la revendication 1, dans laquelle le cancer comprend une tumeur de stade IV.

10. Composition à utiliser selon la revendication 1, dans laquelle le cancer est métastatique.

11. Composition à utiliser selon la revendication 1, dans laquelle le cancer est un cancer choisi dans le groupe constitué du cancer du côlon, du cancer du rectum, du cancer du pancréas et du cancer du foie.

12. Composition à utiliser selon la revendication 1, dans laquelle le cancer est un cancer choisi dans le groupe comprenant : le cancer du pancréas, le cancer de l'utérus, le liposarcome myxoïde, le cancer du côlon et le cancer du foie.

13. Composition à utiliser selon la revendication 1, dans laquelle le sujet n'a pas réagi à un traitement avec un régime chimiothérapeutique comprenant au moins un agent chimiothérapeutique choisi dans le groupe comprenant l'adriamycine, l'ifosfamide, l'étoposide, la vincristine, le gemzar, le taxotère et le Th-302.

14. Composition à utiliser selon la revendication 1, dans laquelle le sujet n'a pas réagi à un traitement avec un régime chimiothérapeutique comprenant au moins un agent chimiothérapeutique choisi dans le groupe comprenant un inhibiteur de topoisomérase I, un inhibiteur de topoisomérase II, un inhibiteur de mitose, un agent alkylant, un composé de platine et un antimétabolite.

15. Composition à utiliser selon l'une quelconque des revendications 1 à 14, dans laquelle la CoQlO est administrée à une dose choisie dans le groupe comprenant au moins 5,6 mg/kg/dose, au moins 11,2 mg/kg/dose, au moins 22,5 mg/kg/dose, au moins 33 mg/kg/dose, au moins 44 mg/kg/dose, au moins 50 mg/kg/dose, au moins 58,7 mg/kg/dose, au moins 75 mg/kg/dose, au moins 78,2 mg/kg/dose, au moins 100 mg/kg/dose, au moins 104,3 mg/kg/dose, au moins 125 mg/kg/dose, au moins 139 mg/kg/dose, au moins 150 mg/kg/dose, et au moins 200 mg/kg/dose.

16. Composition à utiliser selon la revendication 1, dans laquelle au moins 12, 15, 26 ou 33 doses de la composition sont administrées au sujet.

17. Composition à utiliser selon la revendication 1, dans laquelle le sujet n'a pas réagi à 8 régimes chimiothérapeutiques ou moins ou à 5 régimes chimiothérapeutiques ou moins.

18. Composition à utiliser selon la revendication 1, dans laquelle la composition est administrée par injection ou par perfusion.

19. Composition à utiliser selon la revendication 1, dans laquelle le sujet est un être humain.

20. Composition pour l'utilisation selon la revendication 1, dans laquelle la CoQlO est formulée sous forme de nanodispersion.

21. Composition à utiliser selon la revendication 1, dans laquelle la CoQlO est fournie pour une administration intraveineuse dans une formulation de CoQlO comprenant :
une solution aqueuse ;
une CoQlO dispersée dans une nanodispersion de particules ; et
au moins un parmi un agent stabilisateur de dispersion et un réducteur d'opsonisation ;
dans laquelle la nanodispersion de la CoQlO est dispersée en des nanoparticules ayant une taille de particule moyenne inférieure à 200 nm.

22. Composition à utiliser selon la revendication 1, dans laquelle la composition est administrée au sujet avec un agent supplémentaire.

23. Composition à utiliser selon la revendication 22, dans laquelle l'agent supplémentaire est un agent chimiothérapeutique, un agent anticancéreux à petite molécule ou un agent anticancéreux biologique incluant des agents thérapeutiques à la fois à base de protéine et à base d'acide nucléique.

24. Composition à utiliser selon la revendication 1, dans laquelle le cancer est un cancer du pancréas.

25. Composition à utiliser selon la revendication 23, dans laquelle l'agent supplémentaire est la gemcitabine.
